# EUROPEAN PATENT SPECIFICATION

(11) **EP 0 942 996 B1**
(45) Date of publication and mention of the grant of the patent: **03.01.2007**
(21) Application number: 97948587.7
(22) Date of filing: 02.12.1997
(51) Int. Cl.: C12N 15/62, A61K 51/08, A61K 47/48, A61K 38/48, C12N 9/64

(54) **THROMBOLYTIC AGENTS WITH ANTITHROMBOTIC ACTIVITY**
THROMBOLYTISCHE SUBSTANZEN MIT ANTITHROMBOTISCHE AKTIVITÄT
AGENTS THROMBOLYTIQUES A ACTIVITE ANTITHROMBOTIQUE

(30) Priority: 02.12.1996 US 753781
(43) Date of publication of application: 22.09.1999
(73) Proprietor: Diatide, Inc., Londonderry, NH 03053 (US); UNIVERSITY OF SOUTHERN CALIFORNIA, Los Angeles, CA 90033 (US)
(72) Inventor: MARKLAND, Francis, S., Jr., Manhattan Beach, CA 90266 (US); BUSH, Larry, R., Exeter, NH 03833 (US); SWENSON, Stephen, Arcadia, CA 91007 (US); FLORES SANCHEZ, Eladio, CEP-30730-090 Belo Horizonte, MG (BR)
(74) Representative: Dörries, Hans Ulrich
(86) International application number: PCT/US1997/021918
(87) International publication number: WO 1998/024917

(56) References cited:
- WO-A-94/23758
- WO-A-95/25720
- US-A- 4 610 879
- US-A- 5 086 069
- US-A- 5 328 898
- HUA ZI-CHUN ET AL.: "Characterization of a recombinant chimeric plasminogen activator composed of Gly-Pro-Arg-Pro tetrapeptide and truncated urokinase-type plasminogen activator expressed in Escherichia coli." BIOCHEMICAL AND BIOPHYSICAL RESEARCH COMMUNICATIONS, vol. 222, no. 2, 15 May 1996, pages 576-583, XP002058508

### SEQUENCE LISTING

(1) GENERAL INFORMATION:
   (i) APPLICANT: Markland Jr., Francis S.
      Bush, Larry R.
      Swenson, Stephen
      Flores Sanchez, Eladio
   (ii) TITLE OF INVENTION: THROMBOLYTIC AGENTS WITH
      ANTITHROMBOTIC ACTIVITY
   (iii) NUMBER OF SEQUENCES: 54
   (iv) CORRESPONDENCE ADDRESS:
      (A) ADDRESSEE: Diatide, Inc.
      (B) STREET: 9 Delta Drive
      (C) CITY: Londonderry
      (D) STATE: NH
      (E) COUNTRY: USA
      (F) ZIP: 03053
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.30
   (vi) CURRENT APPLICATION DATA:
      (A) APPLICATION NUMBER:
      (B) FILING DATE:
      (C) CLASSIFICATION:
   (vii) PRIOR APPLICATION DATA:
      (A) APPLICATION NUMBER: US 08/753,781
      (B) FILING DATE: 02-DEC-1996
   (viii) ATTORNEY/AGENT INFORMATION:
      (A) NAME: McDaniels, Patricia A.
      (B) REGISTRATION NUMBER: 33,194
      (C) REFERENCE/DOCKET NUMBER: DITI 124.1
   (ix) TELECOMMUNICATION INFORMATION:
      (A) TELEPHONE: 603 437 8970
      (B) TELEFAX: 603 437 8977
(2) INFORMATION FOR SEQ ID NO:1:
   (i) SEQUENCE CHARACTERISTICS:
      (A) LENGTH: 4 amino acids
      (B) TYPE: amino acid
      (C) STRANDEDNESS:
      (D) TOPOLOGY: linear
   (ii) MOLECULE TYPE: peptide
   (xi) SEQUENCE DESCRIPTION: SEQ ID NO:1:
   Gly Pro Arg Pro
   1
(2) INFORMATION FOR SEQ ID NO:2:
   (i) SEQUENCE CHARACTERISTICS:
      (A) LENGTH: 12 amino acids
      (B) TYPE: amino acid
      (C) STRANDEDNESS:
      (D) TOPOLOGY: linear
   (ii) MOLECULE TYPE: peptide
   (xi) SEQUENCE DESCRIPTION: SEQ ID NO:2:
   His His Leu Gly Gly Ala Lys Gln Ala Gly Asp Val
   1 5 10
(2) INFORMATION FOR SEQ ID NO:3:
   (i) SEQUENCE CHARACTERISTICS:
      (A) LENGTH: 13 amino acids
      (B) TYPE: amino acid
      (C) STRANDEDNESS:
      (D) TOPOLOGY: circular
   (ii) MOLECULE TYPE: peptide
   (ix) FEATURE:
      (A) NAME/KEY: Modified-site
      (B) LOCATION: 1
      (D) OTHER INFORMATION: /label= D-Tyrosine
   (ix) FEATURE:
      (A) NAME/KEY: Modified-site
      (B) LOCATION: 2
      (D) OTHER INFORMATION: /label= Apc /note= "Residue X is L-S-(3-aminopropyl)cysteine"
   (ix) FEATURE:
      (A) NAME/KEY: Modified-site
      (B) LOCATION: 1..5
      (D) OTHER INFORMATION: /note= "The side-chain sulfur of cysteine residue 5 is carboxymethylated and esterified to the amino terminal D-tyrosine to form a cyclic peptide "
   (ix) FEATURE:
      (A) NAME/KEY: Modified-site
      (B) LOCATION: 8
      (D) OTHER INFORMATION: /label= Cacm /note= "The side chain thiol group of cysteine 8 is protected by an acetamidomethyl group."
   (ix) FEATURE:
      (A) NAME/KEY: Modified-site
      (B) LOCATION: 10
      (D) OTHER INFORMATION: /label= Cacm /note= "The side chain thiolgroup of cysteine 10 is protected by an acetamidomethyl group"
   (ix) FEATURE:
      (A) NAME/KEY: Modified-site
      (B) LOCATION: 13
      (D) OTHER INFORMATION: /label= amide /note= "The carboxyl terminal cysteine is amidated"
   (xi) SEQUENCE DESCRIPTION: SEQ ID NO:3:
   Tyr Xaa Gly Asp Cys Gly Gly Cys Gly Cys Gly Gly Cys
   1               5                   10
(2) INFORMATION FOR SEQ ID NO:4:
   (i) SEQUENCE CHARACTERISTICS:
      (A) LENGTH: 13 amino acids
      (B) TYPE: amino acid
      (C) STRANDEDNESS:
      (D) TOPOLOGY: circular
   (ii) MOLECULE TYPE: peptide
   (ix) FEATURE:
      (A) NAME/KEY: Modified-site
      (B) LOCATION: 1
      (D) OTHER INFORMATION: /label= D-Tyrosine
   (ix) FEATURE:
      (A) NAME/KEY: Modified-site
      (B) LOCATION: 2
      (D) OTHER INFORMATION: /label= Apc /note= "Residue X is L-S-(3-aminopropyl)cysteine"
   (ix) FEATURE:
      (A) NAME/KEY: Peptide
      (B) LOCATION: 1..5
      (D) OTHER INFORMATION: /note= "The side chain sulfur of cysteine 5 is carboxymethylated and esterified to the amino D-tyrosine to form a cyclic peptide"
   (ix) FEATURE:
      (A) NAME/KEY: Modified-site
      (B) LOCATION: 8
      (D) OTHER INFORMATION: /label= Cacm /note= "The thiol group of cysteine 8 is protected by anacetamidomethyl group"
   (ix) FEATURE:
      (A) NAME/KEY: Modified-site
      (B) LOCATION: 10
      (D) OTHER INFORMATION: /label= Cacm /note= "The thiol group of cysteine 10 is protected by an acetamidomethyl group"
   (ix) FEATURE:
      (A) NAME/KEY: Modified-site
      (B) LOCATION: 13
      (D) OTHER INFORMATION: /label= amide /note= "The carboxyl terminal cysteine is amidated"
   (xi) SEQUENCE DESCRIPTION: SEQ ID NO:4:
   Tyr Xaa Gly Asp Cys Lys Gly Cys Gly Cys Gly Gly Cys
   1               5                   10
(2) INFORMATION FOR SEQ ID NO:5:
   (i) SEQUENCE CHARACTERISTICS:
      (A) LENGTH: 10 amino acids
      (B) TYPE: amino acid
      (C) STRANDEDNESS:
      (D) TOPOLOGY: linear
   (ii) MOLECULE TYPE: peptide
   (ix) FEATURE:
      (A) NAME/KEY: Modified-site
      (B) LOCATION: 1
      (D) OTHER INFORMATION: /label= Cacm /note= "The thiol of cysteine 1 is protected by an acetamidomethyl group"
   (ix) FEATURE:
      (A) NAME/KEY: Modified-site
      (B) LOCATION: 3
      (D) OTHER INFORMATION: /label= Cacm /note= "The thiol of cysteine 3 is protected by an acetamidomethyl group"
   (xi) SEQUENCE DESCRIPTION: SEQ ID NO:5:
   Cys Gly Cys Gly Gly Arg Gly Asp Ser Cys
   1 5 10
(2) INFORMATION FOR SEQ ID NO:6:
   (i) SEQUENCE CHARACTERISTICS:
      (A) LENGTH: 15 amino acids
      (B) TYPE: amino acid
      (C) STRANDEDNESS:
      (D) TOPOLOGY: linear
   (ii) MOLECULE TYPE: peptide
   (ix) FEATURE:
      (A) NAME/KEY: Modified-site
      (B) LOCATION: 1
      (D) OTHER INFORMATION: /label= Cacm /note= "The thiol of cysteine 1 is protected by an acetamidomethyl group"
   (ix) FEATURE:
      (A) NAME/KEY: Modified-site
      (B) LOCATION: 3
      (D) OTHER INFORMATION: /label= Cacm /note= "The thiol of cysteine 3 is protected by an acetamidomethyl group"
   (xi) SEQUENCE DESCRIPTION: SEQ ID NO:6:
   Cys Gly Cys Gly Gly Arg Gly Asp Gly Gly Arg Gly Asp Ser Cys
   1 5 10 15
(2) INFORMATION FOR SEQ ID NO:7:
   (i) SEQUENCE CHARACTERISTICS:
      (A) LENGTH: 20 amino acids
      (B) TYPE: amino acid
      (C) STRANDEDNESS:
      (D) TOPOLOGY: linear
   (ii) MOLECULE TYPE: peptide
   (ix) FEATURE:
      (A) NAME/KEY: Modified-site
      (B) LOCATION: 1
      (D) OTHER INFORMATION: /label= Cacm /note= "The thiol of cysteine 1 is protected by an acetamidomethyl group"
   (ix) FEATURE:
      (A) NAME/KEY: Modified-site
      (B) LOCATION: 3
      (D) OTHER INFORMATION: /label= Cacm /note= "The thiol of cysteine 3 is protected by an acetamidomethyl group"
   (xi) SEQUENCE DESCRIPTION: SEQ ID NO:7:
   Cys Gly Cys Gly Gly Arg Gly Asp Gly Gly Arg Gly Asp Gly Gly Arg
   1 5 10 15
   Gly Asp Ser Cys
   20
(2) INFORMATION FOR SEQ ID NO:8:
   (i) SEQUENCE CHARACTERISTICS:
      (A) LENGTH: 16 amino acids
      (B) TYPE: amino acid
      (C) STRANDEDNESS:
      (D) TOPOLOGY: linear
   (ii) MOLECULE TYPE: peptide
   (ix) FEATURE:
      (A) NAME/KEY: Modified-site
      (B) LOCATION: 1
      (D) OTHER INFORMATION: /label= Cacm /note= "The thiol of cysteine 1 is protected by an acetamidomethyl group"
   (ix) FEATURE:
      (A) NAME/KEY: Modified-site
      (B) LOCATION: 3
      (D) OTHER INFORMATION: /label= Cacm /note= "The thiol of cysteine 3 is protected by an acetamidomethyl group"
   (xi) SEQUENCE DESCRIPTION: SEQ ID NO:8:
   Cys Gly Cys Arg Arg Arg Arg Arg Arg Arg Arg Arg Gly Asp Val Cys
   1 5 10 15
(2) INFORMATION FOR SEQ ID NO:9:
   (i) SEQUENCE CHARACTERISTICS:
      (A) LENGTH: 10 amino acids
      (B) TYPE: amino acid
      (C) STRANDEDNESS:
      (D) TOPOLOGY: linear
   (ii) MOLECULE TYPE: peptide
   (ix) FEATURE:
      (A) NAME/KEY: Modified-site
      (B) LOCATION: 8
      (D) OTHER INFORMATION: /label= Cacm /note= "The thiol of cysteine 8 is protected by an acetamidomethyl group"
   (ix) FEATURE:
      (A) NAME/KEY: Modified-site
      (B) LOCATION: 10
      (D) OTHER INFORMATION: /label= Cacm /note= "The thiol of cysteine 10 is protected by an acetamidomethyl group"
   (ix) FEATURE:
      (A) NAME/KEY: Modified-site
      (B) LOCATION: 10
      (D) OTHER INFORMATION: /label= amide /note= "Carboxyl terminal cysteine 10 is amidated"
   (xi) SEQUENCE DESCRIPTION: SEQ ID NO:9:
   Cys Gly Arg Gly Asp Val Lys Cys Gly Cys
   1 5 10
(2) INFORMATION FOR SEQ ID NO:10:
   (i) SEQUENCE CHARACTERISTICS:
      (A) LENGTH: 9 amino acids
      (B) TYPE: amino acid
      (C) STRANDEDNESS:
      (D) TOPOLOGY: linear
   (ii) MOLECULE TYPE: peptide
   (ix) FEATURE:
      (A) NAME/KEY: Modified-site
      (B) LOCATION: 7
      (D) OTHER INFORMATION: /label= Cacm /note= "The thiol of cysteine 7 is protected by an acetamidomethyl group"
   (ix) FEATURE:
      (A) NAME/KEY: Modified-site
      (B) LOCATION: 9
      (D) OTHER INFORMATION: /label= Cacm /note= "The thiol of cysteine 9 is protected by an acetamidomethyl group"
   (ix) FEATURE:
      (A) NAME/KEY: Modified-site
      (B) LOCATION: 9
      (D) OTHER INFORMATION: /label= amide /note= "Carboxyl terminal cysteine 9 is amidated"
   (xi) SEQUENCE DESCRIPTION: SEQ ID NO:10:
   Cys Gly Arg Gly Asp Val Cys Gly Cys
   1 5
(2) INFORMATION FOR SEQ ID NO:11:
   (i) SEQUENCE CHARACTERISTICS:
      (A) LENGTH: 14 amino acids
      (B) TYPE: amino acid
      (C) STRANDEDNESS:
      (D) TOPOLOGY: linear
   (ii) MOLECULE TYPE: peptide
   (ix) FEATURE:
      (A) NAME/KEY: Modified-site
      (B) LOCATION: 12
      (D) OTHER INFORMATION: /label= Cacm /note= "The thiol of cysteine 12 is protected by an acetamidomethyl group"
   (ix) FEATURE:
      (A) NAME/KEY: Modified-site
      (B) LOCATION: 14
      (D) OTHER INFORMATION: /label= Cacm /note= "The thiol of cysteine 14 is protected by an acetamidomethyl group"
   (ix) FEATURE:
      (A) NAME/KEY: Modified-site
      (B) LOCATION: 14
      (D) OTHER INFORMATION: /label= amide /note= "Carboxyl terminal cysteine 14 is amidated"
   (xi) SEQUENCE DESCRIPTION: SEQ ID NO:11:
   Cys Gly Arg Gly Asp Val Arg Gly Asp Phe Lys Cys Gly Cys
   1 5 10
(2) INFORMATION FOR SEQ ID NO:12:
   (i) SEQUENCE CHARACTERISTICS:
      (A) LENGTH: 13 amino acids
      (B) TYPE: amino acid
      (C) STRANDEDNESS:
      (D) TOPOLOGY: linear
   (ii) MOLECULE TYPE: peptide
   (ix) FEATURE:
      (A) NAME/KEY: Modified-site
      (B) LOCATION: 11
      (D) OTHER INFORMATION: /label= Cacm /note= "The thiol of cysteine 11 is protected by an acetamidomethyl group"
   (ix) FEATURE:
      (A) NAME/KEY: Modified-site
      (B) LOCATION: 13
      (D) OTHER INFORMATION: /label= Cacm /note= "The thiol of cysteine 13 is protected by an acetamidomethyl group"
   (ix) FEATURE:
      (A) NAME/KEY: Modified-site
      (B) LOCATION: 13
      (D) OTHER INFORMATION: /label= amide /note= "Carboxyl terminal cysteine 13 is amidated"
   (xi) SEQUENCE DESCRIPTION: SEQ ID NO:12:
   Cys Gly Arg Gly Asp Val Arg Gly Asp Phe Cys Gly Cys
   1 5 10
(2) INFORMATION FOR SEQ ID NO:13:
   (i) SEQUENCE CHARACTERISTICS:
      (A) LENGTH: 16 amino acids
      (B) TYPE: amino acid
      (C) STRANDEDNESS:
      (D) TOPOLOGY: linear
   (ii) MOLECULE TYPE: peptide
   (ix) FEATURE:
      (A) NAME/KEY: Modified-site
      (B) LOCATION: 1
      (D) OTHER INFORMATION: /label= acetyl /note= "Amino terminal glycine 1 is acetylated"
   (ix) FEATURE:
      (A) NAME/KEY: Modified-site
      (B) LOCATION: 2
      (D) OTHER INFORMATION: /label= Apc /note= "X is L-S-(3-aminopropyl)cysteine"
   (ix) FEATURE:
      (A) NAME/KEY: Modified-site
      (B) LOCATION: 6
      (D) OTHER INFORMATION: /label= Apc /note= "X is L-S-(3-aminopropyl)cysteine"
   (ix) FEATURE:
      (A) NAME/KEY: Modified-site
      (B) LOCATION: 11
      (D) OTHER INFORMATION: /label= Cacm /note= "The thiol of cysteine 11 is protected by an acetamidomethyl group"
   (ix) FEATURE:
      (A) NAME/KEY: Modified-site
      (B) LOCATION: 13
      (D) OTHER INFORMATION: /label= Cacm /note= "The thiol of cysteine 13 is protected by an acetamidomethyl group"
   (ix) FEATURE:
      (A) NAME/KEY: Modified-site
      (B) LOCATION: 16
      (D) OTHER INFORMATION: /label= amide /note= "Carboxyl terminal cysteine 16 is amidated"
   (xi) SEQUENCE DESCRIPTION: SEQ ID NO:13:
   Gly Xaa Gly Asp Val Xaa Gly Asp Phe Lys Cys Gly Cys Gly Gly Cys
   1               5                   10                  15
(2) INFORMATION FOR SEQ ID NO:14:
   (i) SEQUENCE CHARACTERISTICS:
      (A) LENGTH: 16 amino acids
      (B) TYPE: amino acid
      (C) STRANDEDNESS:
      (D) TOPOLOGY: linear
   (ii) MOLECULE TYPE: peptide
   (ix) FEATURE:
      (A) NAME/KEY: Modified-site
      (B) LOCATION: 2
      (D) OTHER INFORMATION: /label= Apc /note= "X is L-S-(3-aminopropyl)cysteine"
   (ix) FEATURE:
      (A) NAME/KEY: Modified-site
      (B) LOCATION: 6
      (D) OTHER INFORMATION: /label= Apc /note= "X is L-S-(3-aminopropyl)cysteine"
   (ix) FEATURE:
      (A) NAME/KEY: Modified-site
      (B) LOCATION: 11
      (D) OTHER INFORMATION: /label= Cacm /note= "The thiol of cysteine 11 is protected by an acetamidomethyl group"
   (ix) FEATURE:
      (A) NAME/KEY: Modified-site
      (B) LOCATION: 13
      (D) OTHER INFORMATION: /label= Cacm /note= "The thiol of cysteine 13 is protected by an acetamidomethyl group"
   (ix) FEATURE:
      (A) NAME/KEY: Modified-site
      (B) LOCATION: 16
      (D) OTHER INFORMATION: /label= amide /note= "Carboxyl terminal cysteine 16 is amidated"
   (xi) SEQUENCE DESCRIPTION: SEQ ID NO:14:
   Gly Xaa Gly Asp Val Xaa Gly Asp Phe Lys Cys Gly Cys Gly Gly Cys
   1 5 10 15
(2) INFORMATION FOR SEQ ID NO:15:
   (i) SEQUENCE CHARACTERISTICS:
      (A) LENGTH: 12 amino acids
      (B) TYPE: amino acid
      (C) STRANDEDNESS:
      (D) TOPOLOGY: linear
   (ii) MOLECULE TYPE: peptide
   (ix) FEATURE:
      (A) NAME/KEY: Modified-site
      (B) LOCATION: 2
      (D) OTHER INFORMATION: /label= Apc /note= "X is L-S-(3-aminopropyl)cysteine"
   (ix) FEATURE:
      (A) NAME/KEY: Modified-site .
      (B) LOCATION: 7
      (D) OTHER INFORMATION: /label= Cacm /note= "The thiol of cysteine 7 is protected by an acetmidomethyl group"
   (ix) FEATURE:
      (A) NAME/KEY: Modified-site
      (B) LOCATION: 9
      (D) OTHER INFORMATION: /label= Cacm /note= "The thiol of cysteine 9 is protected by an acetamidomethyl group"
   (ix) FEATURE:
      (A) NAME/KEY: Modified-site
      (B) LOCATION: 12
      (D) OTHER INFORMATION: /label= amide /note= "Carboxyl terminal cysteine 12 is amidated"
   (xi) SEQUENCE DESCRIPTION: SEQ ID NO:15:
   Gly Xaa Gly Asp Val Lys Cys Gly Cys Gly Gly Cys
   1 5 10
(2) INFORMATION FOR SEQ ID NO:16:
   (i) SEQUENCE CHARACTERISTICS:
      (A) LENGTH: 10 amino acids
      (B) TYPE: amino acid
      (C) STRANDEDNESS:
      (D) TOPOLOGY: linear
   (ii) MOLECULE TYPE: peptide
   (ix) FEATURE:
      (A) NAME/KEY: Modified-site
      (B) LOCATION: 2
      (D) OTHER INFORMATION: /label= Cacm /note= "The thiol of cysteine 2 is protected by an acetamidomethyl group"
   (ix) FEATURE:
      (A) NAME/KEY: Modified-site
      (B) LOCATION: 4
      (D) OTHER INFORMATION: /label= Cacm /note= "The thiol of cysteine 4 is protected by an acetamidomethyl group"
   (xi) SEQUENCE DESCRIPTION: SEQ ID NO:16:
   Cys Cys Gly Cys Gly Gly Arg Gly Asp Ser
   1 5 10
(2) INFORMATION FOR SEQ ID NO:17:
   (i) SEQUENCE CHARACTERISTICS:
      (A) LENGTH: 15 amino acids
      (B) TYPE: amino acid
      (C) STRANDEDNESS:
      (D) TOPOLOGY: linear
   (ii) MOLECULE TYPE: peptide
   (ix) FEATURE:
      (A) NAME/KEY: Modified-site
      (B) LOCATION: 1
      (D) OTHER INFORMATION: /label= acetyl /note= "Amino terminal arginine 1 is acetylated"
   (ix) FEATURE:
      (A) NAME/KEY: Modified-site
      (B) LOCATION: 10
      (D) OTHER INFORMATION: /label= Cacm /note= "The thiol of cysteine 10 is protected by an acetamidomethyl group"
   (ix) FEATURE:
      (A) NAME/KEY: Modified-site
      (B) LOCATION: 12
      (D) OTHER INFORMATION: /label= Cacm /note= "The thiol of cysteine 12 is protected by an acetamidomethyl group"
   (ix) FEATURE:
      (A) NAME/KEY: Modified-site
      (B) LOCATION: 15
      (D) OTHER INFORMATION: /label= amide /note= "Carboxyl terminal cysteine 15 is amidated"
   (xi) SEQUENCE DESCRIPTION: SEQ ID NO:17:
   Arg Arg Ala Arg Gly Asp Asp Leu Asp Cys Gly Cys Gly Gly Cys
   1 5 10 15
(2) INFORMATION FOR SEQ ID NO:18:
   (i) SEQUENCE CHARACTERISTICS:
      (A) LENGTH: 8 amino acids
      (B) TYPE: amino acid
      (C) STRANDEDNESS:
      (D) TOPOLOGY: linear
   (ii) MOLECULE TYPE: peptide
   (ix) FEATURE:
      (A) NAME/KEY: Modified-site
      (B) LOCATION: 8
      (D) OTHER INFORMATION: /label= Cacm /note= "Carboxyl terminal cysteine 8 is protected by an acetamidomethyl group"
   (xi) SEQUENCE DESCRIPTION: SEQ ID NO:18:
   Gly Arg Gly Asp Phe Gly Gly Cys
   1 5
(2) INFORMATION FOR SEQ ID NO:19:
   (i) SEQUENCE CHARACTERISTICS:
      (A) LENGTH: 6 amino acids
      (B) TYPE: amino acid
      (C) STRANDEDNESS:
      (D) TOPOLOGY: linear
   (ii) MOLECULE TYPE: peptide
   (ix) FEATURE:
      (A) NAME/KEY: Modified-site
      (B) LOCATION: 1
      (D) OTHER INFORMATION: /label= mabz /note= "Amino terminal glycine 1 is linked to a (S-benzoyl)-2-mercaptoacetyl group"
   (xi) SEQUENCE DESCRIPTION: SEQ ID NO:19:
   Gly Gly Arg Gly Asp Phe
   1 5
(2) INFORMATION FOR SEQ ID NO:20:
   (i) SEQUENCE CHARACTERISTICS:
      (A) LENGTH: 8 amino acids
      (B) TYPE: amino acid
      (C) STRANDEDNESS:
      (D) TOPOLOGY: linear
   (ii) MOLECULE TYPE: peptide
   (ix) FEATURE:
      (A) NAME/KEY: Modified-site
      (B) LOCATION: 1
      (D) OTHER INFORMATION: /label= Cacm /note= "Amino terminal cysteine 1 is protected by an acetamidomethyl group"
   (xi) SEQUENCE DESCRIPTION: SEQ ID NO:20:
   Cys Gly Gly Gly Arg Gly Asp Phe
   1 5
(2) INFORMATION FOR SEQ ID NO:21:
   (i) SEQUENCE CHARACTERISTICS:
      (A) LENGTH: 7 amino acids
      (B) TYPE: amino acid
      (C) STRANDEDNESS:
      (D) TOPOLOGY: linear
   (ii) MOLECULE TYPE: peptide
   (ix) FEATURE:
      (A) NAME/KEY: Modified-site
      (B) LOCATION: 7
      (D) OTHER INFORMATION: /label= Cacm /note= "Carboxyl terminal cysteine 7 is protected by an acetamidomethyl group"
   (xi) SEQUENCE DESCRIPTION: SEQ ID NO:21:
   Gly Arg Gly Asp Gly Gly Cys
   1 5
(2) INFORMATION FOR SEQ ID NO:22:
   (i) SEQUENCE CHARACTERISTICS:
      (A) LENGTH: 6 amino acids
      (B) TYPE: amino acid
      (C) STRANDEDNESS:
      (D) TOPOLOGY: linear
   (ii) MOLECULE TYPE: peptide
   (ix) FEATURE:
      (A) NAME/KEY: Modified-site
      (B) LOCATION: 1
      (D) OTHER INFORMATION: /label= ma /note= "Amino terminal glycine 1 is linked to a 2-mercaptoacetyl group"
   (xi) SEQUENCE DESCRIPTION: SEQ ID NO:22:
   Gly Gly Arg Gly Asp Phe
   1 5
(2) INFORMATION FOR SEQ ID NO:23:
   (i) SEQUENCE CHARACTERISTICS:
      (A) LENGTH: 7 amino acids
      (B) TYPE: amino acid
      (C) STRANDEDNESS:
      (D) TOPOLOGY: linear
   (ii) MOLECULE TYPE: peptide
   (ix) FEATURE:
      (A) NAME/KEY: Modified-site
      (B) LOCATION: 1
      (D) OTHER INFORMATION: /label= maAcm /note= "Amino terminal glycine 1 is linked to a (S-acetamidomethyl)-2-mercaptoacetyl group"
   (xi) SEQUENCE DESCRIPTION: SEQ ID NO:23:
   Gly Gly Gly Arg Gly Asp Phe
   1   5
(2) INFORMATION FOR SEQ ID NO:24:
   (i) SEQUENCE CHARACTERISTICS:
      (A) LENGTH: 4 amino acids
      (B) TYPE: amino acid
      (C) STRANDEDNESS:
      (D) TOPOLOGY: linear
   (ii) MOLECULE TYPE: peptide
   (ix) FEATURE:
      (A) NAME/KEY: Modified-site
      (B) LOCATION: 1
      (D) OTHER INFORMATION: /label= ma /note= "Amino terminal arginine 1 is linked to a 2-mercaptoacetyl group"
   (xi) SEQUENCE DESCRIPTION: SEQ ID NO:24:
   Arg Gly Asp Phe
   1
(2) INFORMATION FOR SEQ ID NO:25:
   (i) SEQUENCE CHARACTERISTICS:
      (A) LENGTH: 8 amino acids
      (B) TYPE: amino acid
      (C) STRANDEDNESS:
      (D) TOPOLOGY: circular
   (ii) MOLECULE TYPE: peptide
   (ix) FEATURE:
      (A) NAME/KEY: Modified-site
      (B) LOCATION: 1
      (D) OTHER INFORMATION: /label= D-tyrosine
   (ix) FEATURE:
      (A) NAME/KEY: Modified-site
      (B) LOCATION: 2
      (D) OTHER INFORMATION: /label= Apc /note= "X is L-S-(3-aminopropyl)cysteine"
   (ix) FEATURE:
      (A) NAME/KEY: Modified-site
      (B) LOCATION: 1..5
      (D) OTHER INFORMATION: /note= "The side chain sulfur of cysteine 5 is carboxymethylated and esterified to the amino terminal D-tyrosine to form a cyclic peptide "
   (ix) FEATURE:
      (A) NAME/KEY: Modified-site
      (B) LOCATION: 8
      (D) OTHER INFORMATION: /label= amide /note= "Carboxyl terminal cysteine 8 is amidated"
   (xi) SEQUENCE DESCRIPTION: SEQ ID NO:25:
   Tyr Xaa Gly Asp Cys Gly Gly Cys
   1 5
(2) INFORMATION FOR SEQ ID NO:26:
   (i) SEQUENCE CHARACTERISTICS:
      (A) LENGTH: 9 amino acids
      (B) TYPE: amino acid
      (C) STRANDEDNESS:
      (D) TOPOLOGY: linear
   (ii) MOLECULE TYPE: peptide
   (ix) FEATURE:
      (A) NAME/KEY: Modified-site
      (B) LOCATION: 1
      (D) OTHER INFORMATION: /label= D-tyrosine
   (ix) FEATURE:
      (A) NAME/KEY: Modified-site
      (B) LOCATION: 2
      (D) OTHER INFORMATION: /label= Apc /note= "X is L-S-(3-aminopropyl)cysteine"
   (ix) FEATURE:
      (A) NAME/KEY: Modified-site
      (B) LOCATION: 1..5
      (D) OTHER INFORMATION: /note= "The side chain sulfur of cysteine 5 is carboxymethylated and esterified to D-tyrosine 1 to form a cyclic peptide"
   (ix) FEATURE:
      (A) NAME/KEY: Modified-site
      (B) LOCATION: 9
      (D) OTHER INFORMATION: /label= amide /note= "Carboxyl terminal cysteine 9 is amidated"
   (xi) SEQUENCE DESCRIPTION: SEQ ID NO:26:
   Tyr Xaa Gly Asp Cys Gly Gly Gly Cys
   1 5
(2) INFORMATION FOR SEQ ID NO:27:
   (i) SEQUENCE CHARACTERISTICS:
      (A) LENGTH: 10 amino acids
      (B) TYPE: amino acid
      (C) STRANDEDNESS:
      (D) TOPOLOGY: circular
   (ii) MOLECULE TYPE: peptide
   (ix) FEATURE:
      (A) NAME/KEY: Modified-site
      (B) LOCATION: 1
      (D) OTHER INFORMATION: /label= D-tyrosine
   (ix) FEATURE:
      (A) NAME/KEY: Modified-site
      (B) LOCATION: 2
      (D) OTHER INFORMATION: /label= Apc /note= "X is L-S-(3-aminopropyl)cysteine"
   (ix) FEATURE:
      (A) NAME/KEY: Modified-site
      (B) LOCATION: 1..5
      (D) OTHER INFORMATION: /note= "The sidechain sulfur of cysteine 5 is carboxymethylated and esterified to D-tyrosine 1 to form a cyclic peptide"
   (ix) FEATURE:
      (A) NAME/KEY: Modified-site
      (B) LOCATION: 10
      (D) OTHER INFORMATION: /label= amide /note= "Carboxyl terminal cysteine 10 is amidated"
   (xi) SEQUENCE DESCRIPTION: SEQ ID NO:27:
   Tyr Xaa Gly Asp Cys Lys Gly Gly Gly Cys
   1 5 10
(2) INFORMATION FOR SEQ ID NO:28:
   (i) SEQUENCE CHARACTERISTICS:
      (A) LENGTH: 10 amino acids
      (B) TYPE: amino acid
      (C) STRANDEDNESS:
      (D) TOPOLOGY: circular
   (ii) MOLECULE TYPE: peptide
   (ix) FEATURE:
      (A) NAME/KEY: Modified-site
      (B) LOCATION: 1
      (D) OTHER INFORMATION: /label= D-tyrosine
   (ix) FEATURE:
      (A) NAME/KEY: Modified-site
      (B) LOCATION: 2
      (D) OTHER INFORMATION: /label= Apc /note= "X is L-S-(3-aminopropyl)cysteine"
   (ix) FEATURE:
      (A) NAME/KEY: Modified-site
      (B) LOCATION: 1..5
      (D) OTHER INFORMATION: /note= "The side chain sulfur of cysteine 5 is carboxymethylated and esterified to D-tyrosine 1 to form a cyclic peptide"
   (ix) FEATURE:
      (A) NAME/KEY: Modified-site
      (B) LOCATION: 10
      (D) OTHER INFORMATION: /label= amide /note= "Carboxyl terminal cysteine 10 is amidated"
   (xi) SEQUENCE DESCRIPTION: SEQ ID NO:28:
   Tyr Xaa Gly Asp Cys Gly Gly Gly Gly Cys
   1 5 10
(2) INFORMATION FOR SEQ ID NO:29:
   (i) SEQUENCE CHARACTERISTICS:
      (A) LENGTH: 7 amino acids
      (B) TYPE: amino acid
      (C) STRANDEDNESS:
      (D) TOPOLOGY: linear
   (ii) MOLECULE TYPE: peptide
   (xi) SEQUENCE DESCRIPTION: SEQ ID NO:29:
   Gly Gly Arg Gly Asp Ser Cys
   1 5
(2) INFORMATION FOR SEQ ID NO:30:
   (i) SEQUENCE CHARACTERISTICS:
      (A) LENGTH: 12 amino acids
      (B) TYPE: amino acid
      (C) STRANDEDNESS:
      (D) TOPOLOGY: linear
   (ii) MOLECULE TYPE: peptide
   (xi) SEQUENCE DESCRIPTION: SEQ ID NO:30:
   Gly Gly Arg Gly Asp Gly Gly Arg Gly Asp Ser Cys
   1 5 10
(2) INFORMATION FOR SEQ ID NO:31:
   (i) SEQUENCE CHARACTERISTICS:
      (A) LENGTH: 17 amino acids
      (B) TYPE: amino acid
      (C) STRANDEDNESS:
      (D) TOPOLOGY: linear
   (ii) MOLECULE TYPE: peptide
   (xi) SEQUENCE DESCRIPTION: SEQ ID NO:31:
   Gly Gly Arg Gly Asp Gly Gly Arg Gly Asp Gly Gly Arg Gly Asp Ser
   1 5 10 15
   Cys
(2) INFORMATION FOR SEQ ID NO:32:
   (i) SEQUENCE CHARACTERISTICS:
      (A) LENGTH: 13 amino acids
      (B) TYPE: amino acid
      (C) STRANDEDNESS:
      (D) TOPOLOGY: linear
   (ii) MOLECULE TYPE: peptide
   (xi) SEQUENCE DESCRIPTION: SEQ ID NO:32:
   Arg Arg Arg Arg Arg Arg Arg Arg Arg Gly Asp Val Cys
   1 5 10
(2) INFORMATION FOR SEQ ID NO:33:
   (i) SEQUENCE CHARACTERISTICS:
      (A) LENGTH: 7 amino acids
      (B) TYPE: amino acid
      (C) STRANDEDNESS:
      (D) TOPOLOGY: linear
   (ii) MOLECULE TYPE: peptide
   (ix) FEATURE:
      (A) NAME/KEY: Modified-site
      (B) LOCATION: 7
      (D) OTHER INFORMATION: /label= amide /note= "Carboxyl terminal lysine 7 is amidated"
   (xi) SEQUENCE DESCRIPTION: SEQ ID NO:33:
   Cys Gly Arg Gly Asp Val Lys
   1 5
(2) INFORMATION FOR SEQ ID NO:34:
   (i) SEQUENCE CHARACTERISTICS:
      (A) LENGTH: 6 amino acids
      (B) TYPE: amino acid
      (C) STRANDEDNESS:
      (D) TOPOLOGY: linear
   (ii) MOLECULE TYPE: peptide
   (ix) FEATURE:
      (A) NAME/KEY: Modified-site
      (B) LOCATION: 6
      (D) OTHER INFORMATION: /label= amide /note= "Carboxyl terminal valine 6 is amidated"
   (xi) SEQUENCE DESCRIPTION: SEQ ID NO:34:
   Cys Gly Arg Gly Asp Val
   1 5
(2) INFORMATION FOR SEQ ID NO:35:
   (i) SEQUENCE CHARACTERISTICS:
      (A) LENGTH: 11 amino acids
      (B) TYPE: amino acid
      (C) STRANDEDNESS:
      (D) TOPOLOGY: linear
   (ii) MOLECULE TYPE: peptide
   (ix) FEATURE:
      (A) NAME/KEY: Modified-site
      (B) LOCATION: 11
      (D) OTHER INFORMATION: /label= amide /note= "Carboxyl terminal lysine 11 is amidated"
   (xi) SEQUENCE DESCRIPTION: SEQ ID NO:35:
   Cys Gly Arg Gly Asp Val Arg Gly Asp Phe Lys
   1 5 10
(2) INFORMATION FOR SEQ ID NO:36:
   (i) SEQUENCE CHARACTERISTICS:
      (A) LENGTH: 10 amino acids
      (B) TYPE: amino acid
      (C) STRANDEDNESS:
      (D) TOPOLOGY: linear
   (ii) MOLECULE TYPE: peptide
   (ix) FEATURE:
      (A) NAME/KEY: Modified-site
      (B) LOCATION: 10
      (D) OTHER INFORMATION: /label= amide /note= "Carboxyl terminal phenylalanine 10 is amidated"
   (xi) SEQUENCE DESCRIPTION: SEQ ID NO:36:
   Cys Gly Arg Gly Asp Val Arg Gly Asp Phe
   1 5 10
(2) INFORMATION FOR SEQ ID NO:37:
   (i) SEQUENCE CHARACTERISTICS:
      (A) LENGTH: 13 amino acids
      (B) TYPE: amino acid
      (C) STRANDEDNESS:
      (D) TOPOLOGY: linear
   (ii) MOLECULE TYPE: peptide
   (ix) FEATURE:
      (A) NAME/KEY: Modified-site
      (B) LOCATION: 1
      (D) OTHER INFORMATION: /label= acetyl /note= "Amino terminal glycine 1 is acetylated"
   (ix) FEATURE:
      (A) NAME/KEY: Modified-site
      (B) LOCATION: 2
      (D) OTHER INFORMATION: /label= Apc /note= "X is L-S-(3-aminopropyl)cysteine"
   (ix) FEATURE:
      (A) NAME/KEY: Modified-site
      (B) LOCATION: 6
      (D) OTHER INFORMATION: /label= Apc /note= "X is L-S-(3-aminopropyl)cysteine"
   (ix) FEATURE:
      (A) NAME/KEY: Modified-site
      (B) LOCATION: 13
      (D) OTHER INFORMATION: /label= amide /note= "Carboxyl terminal cysteine 13 is amidated"
   (xi) SEQUENCE DESCRIPTION: SEQ ID NO:37:
   Gly Xaa Gly Asp Val Xaa Gly Asp Phe Lys Gly Gly Cys
   1 5 10
(2) INFORMATION FOR SEQ ID NO:38:
   (i) SEQUENCE CHARACTERISTICS:
      (A) LENGTH: 13 amino acids
      (B) TYPE: amino acid
      (C) STRANDEDNESS:
      (D) TOPOLOGY: linear
   (ii) MOLECULE TYPE: peptide
   (ix) FEATURE:
      (A) NAME/KEY: Modified-site
      (B) LOCATION: 2
      (D) OTHER INFORMATION: /label= Apc /note= "X is L-S-(3-aminopropyl)cysteine"
   (ix) FEATURE:
      (A) NAME/KEY: Modified-site
      (B) LOCATION: 6
      (D) OTHER INFORMATION: /label= Apc /note= "X is L-S-(3-aminopropyl)cysteine"
   (ix) FEATURE:
      (A) NAME/KEY: Modified-site
      (B) LOCATION: 13
      (D) OTHER INFORMATION: /label= amide /note= "Carboxyl terminal cysteine 13 is amidated"
   (xi) SEQUENCE DESCRIPTION: SEQ ID NO:38:
   Gly Xaa Gly Asp Val Xaa Gly Asp Phe Lys Gly Gly Cys
   1 5 10
(2) INFORMATION FOR SEQ ID NO:39:
   (i) SEQUENCE CHARACTERISTICS:
      (A) LENGTH: 9 amino acids
      (B) TYPE: amino acid
      (C) STRANDEDNESS:
      (D) TOPOLOGY: linear
   (ii) MOLECULE TYPE: peptide
   (ix) FEATURE:
      (A) NAME/KEY: Modified-site
      (B) LOCATION: 2
      (D) OTHER INFORMATION: /label= Apc /note= "X is L-S-(3-aminopropyl)cysteine"
   (ix) FEATURE:
      (A) NAME/KEY: Modified-site
      (B) LOCATION: 9
      (D) OTHER INFORMATION: /label= amide /note= "Carboxyl terminal cysteine 9 is amidated"
   (xi) SEQUENCE DESCRIPTION: SEQ ID NO:39:
   Gly Xaa Gly Asp Val Lys Gly Gly Cys
   1 5
(2) INFORMATION FOR SEQ ID NO:40:
   (i) SEQUENCE CHARACTERISTICS:
      (A) LENGTH: 7 amino acids
      (B) TYPE: amino acid
      (C) STRANDEDNESS:
      (D) TOPOLOGY: linear
   (ii) MOLECULE TYPE: peptide
   (xi) SEQUENCE DESCRIPTION: SEQ ID NO:40:
   Cys Gly Gly Arg Gly Asp Ser
   1 5
(2) INFORMATION FOR SEQ ID NO:41:
   (i) SEQUENCE CHARACTERISTICS:
      (A) LENGTH: 12 amino acids
      (B) TYPE: amino acid
      (C) STRANDEDNESS:
      (D) TOPOLOGY: linear
   (ii) MOLECULE TYPE: peptide
   (ix) FEATURE:
      (A) NAME/KEY: Modified-site
      (B) LOCATION: 1
      (D) OTHER INFORMATION: /label= acetyl /note= "Amino terminal arginine 1 is acetylated"
   (ix) FEATURE:
      (A) NAME/KEY: Modified-site
      (B) LOCATION: 12
      (D) OTHER INFORMATION: /label= amide /note= "Carboxyl terminal cysteine 12 is amidated"
   (xi) SEQUENCE DESCRIPTION: SEQ ID NO:41:
   Arg Arg Ala Arg Gly Asp Asp Leu Asp Gly Gly Cys
   1 5 10
(2) INFORMATION FOR SEQ ID NO:42:
   (i) SEQUENCE CHARACTERISTICS:
      (A) LENGTH: 13 amino acids
      (B) TYPE: amino acid
      (C) STRANDEDNESS:
      (D) TOPOLOGY: linear
   (ii) MOLECULE TYPE: peptide
   (xi) SEQUENCE DESCRIPTION: SEQ ID NO:42:
   Cys Lys Arg Ala Arg Gly Asp Asp Met Asp Asp Tyr Cys
   1 5 10
(2) INFORMATION FOR SEQ ID NO:43:
   (i) SEQUENCE CHARACTERISTICS:
      (A) LENGTH: 7 amino acids
      (B) TYPE: amino acid
      (C) STRANDEDNESS:
      (D) TOPOLOGY: linear
   (ii) MOLECULE TYPE: peptide
   (ix) FEATURE:
      (A) NAME/KEY: Modified-site
      (B) LOCATION: 1
      (D) OTHER INFORMATION: /label= mmp /note= "Amino terminal glycine 1 is linked to a 2-mercapto-2-methylpropionyl group"
   (xi) SEQUENCE DESCRIPTION: SEQ ID NO:43:
   Gly Gly Gly Arg Gly Asp Phe
   1 5
(2) INFORMATION FOR SEQ ID NO:44:
   (i) SEQUENCE CHARACTERISTICS:
      (A) LENGTH: 4 amino acids
      (B) TYPE: amino acid
      (C) STRANDEDNESS:
      (D) TOPOLOGY: linear
   (ii) MOLECULE TYPE: peptide
   (ix) FEATURE:
      (A) NAME/KEY: Modified-site
      (B) LOCATION: 1
      (D) OTHER INFORMATION: /label= acetyl /note= "Amino terminal arginine 1 is acetylated"
   (ix) FEATURE:
      (A) NAME/KEY: Modified-site
      (B) LOCATION: 4
      (D) OTHER INFORMATION: /label= amide /note= "Carboxyl terminal cysteine 4 is amidated"
   (xi) SEQUENCE DESCRIPTION: SEQ ID NO:44:
   Arg Gly Asp Cys
   1
(2) INFORMATION FOR SEQ ID NO:45:
   (i) SEQUENCE CHARACTERISTICS:
      (A) LENGTH: 5 amino acids
      (B) TYPE: amino acid
      (C) STRANDEDNESS:
      (D) TOPOLOGY: linear
   (ii) MOLECULE TYPE: peptide
   (xi) SEQUENCE DESCRIPTION: SEQ ID NO:45:
   Cys Arg Gly Asp Cys
   1 5
(2) INFORMATION FOR SEQ ID NO:46:
   (i) SEQUENCE CHARACTERISTICS:
      (A) LENGTH: 8 amino acids
      (B) TYPE: amino acid
      (C) STRANDEDNESS:
      (D) TOPOLOGY: linear
   (ii) MOLECULE TYPE: peptide
   (xi) SEQUENCE DESCRIPTION: SEQ ID NO:46:
   Cys Gly Gly Gly Arg Gly Asp Phe
   1 5
(2) INFORMATION FOR SEQ ID NO:47:
   (i) SEQUENCE CHARACTERISTICS:
      (A) LENGTH: 9 amino acids
      (B) TYPE: amino acid
      (C) STRANDEDNESS:
      (D) TOPOLOGY: linear
   (ii) MOLECULE TYPE: peptide
   (xi) SEQUENCE DESCRIPTION: SEQ ID NO:47:
   Gly Arg Gly Asp Gly Gly Gly Gly Cys
   1 5
(2) INFORMATION FOR SEQ ID NO:48:
   (i) SEQUENCE CHARACTERISTICS:
      (A) LENGTH: 7 amino acids
      (B) TYPE: amino acid
      (C) STRANDEDNESS:
      (D) TOPOLOGY: linear
   (ii) MOLECULE TYPE: peptide
   (xi) SEQUENCE DESCRIPTION: SEQ ID NO:48:
   Gly Arg Gly Asp Gly Gly Cys
   1 5
(2) INFORMATION FOR SEQ ID NO:49:
   (i) SEQUENCE CHARACTERISTICS:
      (A) LENGTH: 7 amino acids
      (B) TYPE: amino acid
      (C) STRANDEDNESS:
      (D) TOPOLOGY: linear
   (ii) MOLECULE TYPE: peptide
   (ix) FEATURE:
      (A) NAME/KEY: Modified-site
      (B) LOCATION: 1
      (D) OTHER INFORMATION: /label= maAcm /note= "Amino terminal glycine 1 is linked to a (S-acetamido)-2-mercaptoacetyl group"
   (xi) SEQUENCE DESCRIPTION: SEQ ID NO:49:
   Gly Gly Gly Arg Gly Asp Phe
   1 5
(2) INFORMATION FOR SEQ ID NO:50:
   (i) SEQUENCE CHARACTERISTICS:
      (A) LENGTH: 7 amino acids
      (B) TYPE: amino acid
      (C) STRANDEDNESS:
      (D) TOPOLOGY: linear
   (ii) MOLECULE TYPE: peptide
   (ix) FEATURE:
      (A) NAME/KEY: Modified-site
      (B) LOCATION: 1
      (D) OTHER INFORMATION: /label= acetyl /note= "Amino terminal cysteine 1 is acetylated"
   (ix) FEATURE:
      (A) NAME/KEY: Modified-site
      (B) LOCATION: 4
      (D) OTHER INFORMATION: /label= Apc /note= "X is L-S-(3-aminopropyl)cysteine"
   (xi) SEQUENCE DESCRIPTION: SEQ ID NO:50:
   Cys Asn Pro Xaa Gly Asp Cys
   1               5
(2) INFORMATION FOR SEQ ID NO:51:
   (i) SEQUENCE CHARACTERISTICS:
      (A) LENGTH: 13 amino acids
      (B) TYPE: amino acid
      (C) STRANDEDNESS:
      (D) TOPOLOGY: linear
   (ii) MOLECULE TYPE: peptide
   (xi) SEQUENCE DESCRIPTION: SEQ ID NO:51:
   Cys Arg Ile Ala Arg Gly Asp Trp Asn Asp Asp Tyr Cys
   1               5                   10
(2) INFORMATION FOR SEQ ID NO:52:
   (i) SEQUENCE CHARACTERISTICS:
      (A) LENGTH: 28 amino acids
      (B) TYPE: amino acid
      (C) STRANDEDNESS:
      (D) TOPOLOGY: linear
   (ii) MOLECULE TYPE: peptide
   (xi) SEQUENCE DESCRIPTION: SEQ ID NO:52:
   Cys Lys Phe Phe Ala Arg Thr Val Cys Arg Ile Ala Arg Gly Asp Trp
   1 5 10 15
   Asn Asp Asp Tyr Cys Thr Gly Lys Ser Ser Asp Cys
   20 25
(2) INFORMATION FOR SEQ ID NO:53:
   (i) SEQUENCE CHARACTERISTICS:
      (A) LENGTH: 16 amino acids
      (B) TYPE: amino acid
      (C) STRANDEDNESS:
      (D) TOPOLOGY: linear
   (ii) MOLECULE TYPE: peptide
   (xi) SEQUENCE DESCRIPTION: SEQ ID NO:53:
   Lys Tyr Gly Gly His His Leu Gly Gly Ala Lys Gln Ala Gly Asp Val
   1 5 10 15
(2) INFORMATION FOR SEQ ID NO:54:
   (i) SEQUENCE CHARACTERISTICS:
      (A) LENGTH: 9 amino acids
      (B) TYPE: amino acid
      (C) STRANDEDNESS:
      (D) TOPOLOGY: circular
   (ii) MOLECULE TYPE: peptide
   (ix) FEATURE:
      (A) NAME/KEY: Modified-site
      (B) LOCATION: 1
      (D) OTHER INFORMATION: /label= D-tyrosine
   (ix) FEATURE:
      (A) NAME/KEY: Modified-site
      (B) LOCATION: 2
      (D) OTHER INFORMATION: /label= Amp /note= "X is 4-amidinophenylalanine"
   (ix) FEATURE:
      (A) NAME/KEY: Modified-site
      (B) LOCATION: 1..5
      (D) OTHER INFORMATION: /note= "The side chain sulfur of cysteine 5 is carboxymethylated and esterified to D-tyrosine 1 to form a cyclic peptide"
   (ix) FEATURE:
      (A) NAME/KEY: Modified-site
      (B) LOCATION: 9
      (D) OTHER INFORMATION: /label= amide /note= "Carboxyl terminal glycine 9 is amidated"
   (xi) SEQUENCE DESCRIPTION: SEQ ID NO:54:
   Tyr Xaa Gly Asp Cys Lys Gly Cys Gly
   1 5

## Description

### BACKGROUND OF THE INVENTION

This invention relates to thrombolytic agents and methods for making and using such agents. Specifically, the invention relates to thrombolytic agents comprising a thrombolytic proteinase covalently linked to a targeting compound that specifically binds to blood clots (thrombi) *in vivo.* The thrombolytic agents of the invention are useful for eliminating thrombi *in vivo* to alleviate pathological conditions caused thereby.

Thrombosis and thromboembolism, *i.e.,* the occurrence of occlusive thrombi in the vasculature of human patients, poses a significant clinical problem and is a significant cause of morbidity and mortality. Arterial thrombi may occur at atherosclerotic plaque and are responsible for myocardial infarction (MI) and cerebral ischemia (stroke). Venous thrombi can cause deep vein thrombosis (DVT) and pulmonary embolism (PE). The magnitude of the clinical challenge created by thrombosis is reflected in morbidity and mortality statistics. One of the leading causes of death in men over the age of 50 is acute MI, and stroke remains a debilitating and unpredictable disease. It has been estimated that in the U.S. approximately 5 million patients experience one or more episodes of DVT per year and that over 500,000 cases of PE occur, resulting in 100,000 deaths (Seabold, Society of Nuclear Medicine Annual Meeting 1990).

All thrombi contain blood cells, fibrin, and coagulation proteins, with the precise composition of each thrombus varying according to thrombus location and etiology. For example, venous thrombi are generally fibrin rich and platelet poor, also containing red blood cells. In comparison, arterial thrombi are composed mainly of platelet aggregates interconnected by thin fibrin strands. As another example, thrombi that form after blood flow is restored in an occluded artery consist almost entirely of activated platelets, coagulation proteins, and a relatively small amount of fibrin. Exemplary coagulation proteins that may be found in thrombi include thrombin, factor XIII, α₂-antiplasmin, von Willebrand factor, fibronectin, fibrinogen, plasmin, and the like.

Thrombus formation is believed to occur through activation of the complex blood coagulation cascade at a site of a vascular injury. The injury exposes the subendothelium of the blood vessel, which triggers platelet activation and subsequent platelet adherence to the vascular subendothelium. When activated, platelets degranulate, that is, secrete substances which attract other platelets to the site of the injury such as epinephrine, adenosine diphosphate, thromboxane A₂, and serotinin. Activated platelets also secrete thrombin, a multi-substrate protease that promotes more platelets to become activated and to aggregate. Thrombin also converts fibrinogen to fibrin, the structural scaffold of the thrombus. Another enzyme, Factor XIIIa, crosslinks fibrin to fortify the structure of the clot. In normal hemostasis, the thrombus is dissolved through the action of the proteolytic enzyme plasmin on the crosslinked fibrin, through activation of the plasmin precursor plasminogen. When normal hemostasis is disturbed, the clot is not dissolved and may form a local occlusion or embolism. Alternatively, the thrombus may detach from its site of formation and travel through the cardiovascular system to form an embolism at a distant site.

Anticoagulant therapy can effectively treat thrombosis in many cases, if applied early enough. However, such treatment is associated with risks, for example, internal bleeding. In addition, particularly in arterial thrombi, rethrombosis and reocclusion of blood vessels at the thrombus site is a significant (10-30%) clinical outcome. Even using the most advanced methodologies currently available, 25-30% of acute MI patients fail to restore perfusion to the thrombus-occluded coronary artery that is the proximal cause of the heart attack.

Plasminogen-activating enzymes such as streptokinase, urokinase, and tissue plasminogen activator (tPA) are currently in clinical use for dissolving arterial thrombi, particularly thrombi found in coronary arteries. These enzymes act by generating plasmin. While plasmin is effective in dissolving crosslinked fibrin in clots, the proteolytic actions of plasmin on circulating proteins such as fibrinogen can cause serious systemic side effects such as bleeding at noncoronary locations. Moreover, large doses of the plasminogen-activating enzymes are necessary for prompt dissolution of thrombi. Another disadvantage of the plasminogen-activating enzymes lies in their ability to activate thrombin, thus increasing the likelihood of rethrombosis. A further disadvantage of streptokinase is that it is a bacterial product, and thus streptokinase induces a long lasting antibody response, limiting its utility for a second use if additional thrombosis occurs.

A number of pharmaceutical agents have been developed to attempt targeted delivery of thrombolytic agents to thrombus sites *in vivo* and thus to limit the side effects of the known agents. In particular, conjugates have been made between thrombus component-specific antibodies and plasminogen activating enzymes. Such conjugates have employed monoclonal antibodies against the glycoprotein IIb/IIIa integrin (GPIIb/IIIa) receptor present on platelet membranes or monoclonal antibodies against a coagulation protein. One such antibody has been approved for clinical use. Of course, use of antibodies, even humanized antibodies, can cause undesirable immunological side effects. In addition, the approved monoclonal antibody has been associated with thrombocytopenia and prolonged bleeding times in some patients.

A large number of proposed antithrombotic agents have employed peptides containing the amino acid motif -Arg-Gly-Asp (RGD), which represents the fibrinogen recognition site for the GPIIb/IIIa receptor. Clinical studies have indicated that one such peptide agent was therapeutically effective only for a subset of patients. Peptidomimetic agents based on the RGD motif are also in early clinical studies. Bleeding has been associated with all of the RGD motif-based agents, and none of these agents have been approved for human use.

U.S.Pat.No. 4,610,879 discloses fibrolase, a zinc metalloproteinase purified from the venom of the Southern Copperhead snake *(Agkistrodon contortrix contortrix)* which has direct proteolytic activity for fibrin. Fibrolase does not activate plasminogen and thus does not demonstrate the hemorrhagic activity of the plasminogen activating thrombolytic agents. However, as disclosed in U.S.Pat.No. 5,260,060, fibrolase exhibits general proteolytic activity which may preclude use of high dosages or long-term administration. U.S.Pat.No. 5,260,060 discloses additional zinc metalloproteinases from the venom of the Mexican west coast rattlesnake *(Crotalus basiliscus basiliscus)* which act directly on fibrin but which are less active as general proteinases than fibrolase.

A need exists for thrombolytic agents that can specifically target thrombus sites *in vivo* and thereby reduce or eliminate deleterious systemic side effects of known agents.

### SUMMARY OF THE INVENTION

The present invention provides thrombolytic agents which are specifically targeted to thrombus sites *in vivo* and which have minimal hemorrhagic side effects. The thrombolytic agents of the invention further have minimal side effects related to non-specific proteolysis. The preferred thrombolytic agents of the invention do not appreciably activate thrombin, and thus do not contribute to rethrombosis after successful treatment of an acute thrombotic episode. The thrombolytic agents of the invention are covalently linked conjugates between a proteolytic enzyme that acts on a component of a thrombus and a targeting compound that specifically binds at the thrombus site.

In one embodiment, the invention provides a thrombolytic agent comprising a thrombolytic metalloproteinase covalently linked to a targeting compound capable of specifically binding to a component of a thrombus.

The invention also provides methods for making the thrombolytic agents of the invention by a chemical synthesis using a crosslinking agent or, when the targeting compound is a peptide, by recombinant techniques.

The invention also relates to a method for using the thrombolytic agents of the invention for dissolving clots at pathological sites *in vivo,* comprising the step of administering an effective therapeutic amount of the thrombolytic agent of the invention.

The invention also relates to a method whereby targeting and dissolution of the blood clot can be monitored, for example, by scintigraphy. This method comprises the steps of administering an effective therapeutic amount of a radiolabeled thrombolytic agent of the invention, detecting the radiation emitted by the radiolabel localized at the thrombus site, and observing diminution of the radiation as the thrombus is dissolved by the thrombolytic agent.

### BRIEF DESCRIPTION OF THE DRAWINGS

Figure 1 illustrates the reaction scheme for the conjugation of fibrolase with a targeting peptide of the invention using N-(γ-maleimidobutyryloxy) sulfosuccinimide ester (S-GMBS).
Figure 2 shows the chemical structure of a fluorescent detection reagent (SAMSA fluorescein) used to determine the extent of chemical adduct formation between fibrolase and S-GMBS.
Figure 3 illustrates the reaction scheme for the conjugation of fibrolase with a targeting peptide of the invention using N-succinimidyl-3-(2-pyridyldithio) propionate (SPDP).
Figure 4 illustrates the reaction scheme for the conjugation of fibrolase with a targeting peptide of the invention using sulfosuccinimidyl-6-(α-methyl-α-(2-pyridyldithio) toluamido) hexonate (S-SMPT).
Figure 5 shows the results of HPLC purification of SPDP crosslinked fibrolase:peptide conjugates. Panel A illustrates the elution profile of unconjugated fibrolase isoform 2 control; Panel B shows the elution profile of unconjugated peptide; Panel C shows the elution profile of SPDP-linked fibrolase; and Panel D shows the elution profile of fibrolase:peptide conjugates crosslinked with SPDP.
Figure 6 shows the results of amino acid composition analysis of the peptide CH₂CO-Y_{D}.Amp.GDCKGCG.amide (Panel A), fibrolase (Panel B), and a conjugate of fibrolase: CH₂CO-Y_{D}.Amp.GDCKGCG.amide crosslinked with SPDP (Panel C).

### DETAILED DESCRIPTION OF THE PREFERRED EMBODIMENTS

The thrombolytic agents of the present invention possess several advantages over thrombolytic agents known in the prior art. The targeting peptides of the thrombolytic agents of the invention minimize the amount of thrombolytic proteinase required to effectively dissolve thrombi. The minimal dosage of thrombolytic proteinase results in fewer systemic side effects than are observed with currently available thrombolytic agents. The thrombolytic agents of the invention may further be designed to act only on fibrin, through the choice of proteinase, thereby minimizing the risk of systemic side effects and rethrombosis.

For the purposes of this invention, the term "thrombolytic agent" encompasses any enzymatic or chemical agent that disrupts, dissolves, digests, dissociates, lyses, reduces, eliminates or ameliorates (collectively termed "thrombolytic properties") thrombi and blood clots in arteries, veins, arterioles, capillaries and other vascular structures in an animal body, as well as in various tissue sites including but not limited to brain, lung, kidney, skin, heart, and deep veins of the leg.

For the purposes of this invention, the term "thrombolytic proteinase" is intended to encompass a metalloproteinase Metalloproteinases derived from venoms, in particular, from snake venoms, may also be used in the thrombolytic agents of the invention. Use of a metalloproteinase minimizes hemorrhagic side effects and maximizes *in vivo* efficacy, since metalloproteinases are not inhibited by serpins and other circulating inhibitors of serine protease activity found in animal circulation. The thrombolytic agent of the invention may comprise a fragment of a thrombolytic proteinase which retains proteolytic activity for a thrombus component. Preferably, the proteinase employed in the thrombolytic agent of the invention is a zinc metalloproteinase such as the snake venom-derived proteinases described in U.S.Pat.Nos. 4,610,879 and 5,260,060. More preferably, the thrombolytic proteinase employed in the invention is fibrolase isolated from *A. contortrix contortrix* venom and identified as EC 3.4.24.72. Most preferably, the thrombolytic proteinase employed in the invention is prepared using recombinant genetic technology, for example, as disclosed in European Patent Application No. 323722.

Any peptide capable of specifically binding to a thrombus *in vivo* may be employed as a targeting peptide in accordance with the present invention. For the purposes of this invention, the term "targeting peptide includes peptides having a biological affinity for specific components of tissues and other biological structures in a thrombus, including most preferably a coagulation protein, fibrin, and platelets. In other embodiments, the targeting peptide may specifically bind to any other component of a thrombus. The term "targeting" as defined herein specifically excludes hirudin and hirudin analogs, proteins such as antibodies and large protein fragments such as antibody Fc fragments, Fab' fragments, and single chain antibody fragments. The targeting peptide preferably comprises from about 3 to about 100 amino acids, more preferably about 4 to about 50 amino acids, and most preferably from about 4 to about 20 amino acids.

The thrombolytic agent of the invention may comprise more than one targeting compound covalently linked to the thrombolytic proteinase, to enhance the affinity of the agent for thrombi. In this embodiment, at least two targeting compounds are covalently linked to the proteinase. The linkages of the targeting compounds to the proteinase in this embodiment may occur at any site or sites on the proteinase molecule that does not substantially reduce the thrombolytic activity of the proteinase. Covalent linkage of the targeting peptides to the proteinase may be configured in any way, *e.g*., in a configuration such as targeting peptide proteinase-targeting peptide

Alternatively, the targeting peptides may be configured to extend from a single site on the proteinase to form a molecular "hook", *e.g.*, in a configuration such as targeting peptide targeting peptide proteinase Targeting molecules having different binding specificities may be employed in this embodiment, or a multiplicity of targeting molecules having the same binding specificity may be employed.

In one embodiment, the targeting peptide of the thrombolytic agent of the invention specifically binds to platelets. In this embodiment, the targeting peptide preferably specifically binds to the platelet GPIIb/IIIa receptor which is present on platelets. For example, GPIIb/IIIa receptor specific guanidine derivatives such as those disclosed in U.S.Pat.No. 5,086,069 may be employed as targeting compounds in the thrombolytic agents of the invention. Similarly, GPIIb/IIIa receptor specific tyrosine derivatives such as those described in EP 478328 and in Hartman *et al.* (1992) *J. Med. Chem.* **35,** 4640-4642 may be used as targeting compounds in accordance with the invention. The targeting peptide may be a linear peptide ligand for a GPIIb/IIIa receptor or a cyclic peptide ligand for a GPIIb/IIIa receptor. Preferably said targeting peptide has an amino acid sequence comprising the sequence -XGD-, wherein X is any amino acid having a sidechain comprising an amino group, a guanidino group or an amidino group. In the platelet-specific embodiment, the targeting compound may optionally be capable of inhibiting platelet aggregation and platelet-fibrinogen interactions. When the targeting compound is capable of inhibiting platelet aggregation, the compound is capable of inhibiting human platelet aggregation in platelet-rich plasma by 50% (*i.e.,* has an IC₅₀ for platelet aggregation) when present at a concentration of no more than 1 µM, more preferably no more than 0.3 µM, and most preferably no more than 0.1 µM.

In another embodiment, the targeting peptide specifically binds to fibrin. As the fibrin-specific targeting compound is a peptide, a peptide ligand for a polymerization site of fibrin is preferred. Specific ligands for a polymerization site of fibrin include, for example, peptides comprising multiple copies of the amino acid sequence (Gly-Pro-Arg-Pro).

Examples of targeting peptides useful as components of the thrombolytic agents of the invention include peptides comprising the amino acid sequence -XGD-, wherein X is an amino acid comprising an amino group, a guanidino group or an amidino group in its sidechain, and peptides comprisig the amino acid sequence -GPRP- (SEQ ID NO:1), and including but not limited to peptides having the following formulae:
HHLGGAKQAGDV (SEQ ID NO:2),
CH₂CO.Y_{D}.Apc.GDCGGC_{Acm}GC_{Acm}GGC.amide (SEQ ID NO:3),
CH₂CO.Y_{D}.Apc.GDCKGC_{Acm}GC_{Acm}GGC.amide (SEQ ID NO:4),
C_{Acm}GC_{Acm}GGRGDSC (SEQ ID NO:5),
C_{Acm}GC_{Acm}GGRGDGGRGDSC (SEQ ID NO:6),
C_{Acm}GC_{Acm}GGRGDGGRGDGGRGDSC (SEQ ID NO:7),
C_{Acm}GC_{Acm}RRRRRRRRRGDVC (SEQ ID NO:8),
CGRGDVKC_{Acm}GC_{Acm}.amide (SEQ ID NO:9),
CGRGDVC_{Acm}GC_{Acm}.amide (SEQ ID NO: 10),
CGRGDVRGDFKC_{Acm}GC_{Acm}.amide (SEQ ID NO:11),
CGRGDVRGDFC_{Acm}GC_{Acm}.amide (SEQ ID NO:12),
*acetyl*-G.Apc.GDV.Apc.GDFKC_{Acm}GC_{Acm}.GGCamide (SEQ ID NO:13),
G.Apc.GDV.Apc.GDFKC_{Acm}GC_{Acm}.GGCamide (SEQ ID NO:14),
G.Apc.GDVKC_{Acm}GC_{Acm}GGC.amide (SEQ ID NO:15),
CC_{Acm}GC_{Acm}GGRGDS (SEQ ID NO:16),
*acetyl*-RRARGDDLDC_{Acm}GC_{Acm}GGC.amide (SEQ ID NO:17),
GRGDFGGC_{Acm} (SEQ ID NO:18),
*ma_{Bz}*-GGRGDF (SEQ ID NO:19),
C_{Acm}GGGRGDF (SEQ ID NO:20),
GRGDGGC_{Acm} (SEQ ID NO:21),
*ma*-GGRGDF (SEQ ID NO:22),
*ma_{Acm}*-GGGRGDF (SEQ ID NO:23),
*ma*-RGDF (SEQ ID NO:24),
*ma*-RGD,
CH₂CO.Y_{D}.Apc.GDCGGC.amide (SEQ ID NO:25),
CH₂CO.Y_{D}.Apc.GDCGGGC.amide (SEQ ID NO:26),
CH₂CO.Y_{D}.Apc.GDCKGGGC.amide (SEQ ID NO:27)
CH₂CO.Y_{D}.Apc.GDCGGGGC.amide (SEQ ID NO:28),
GGRGDSC (SEQ ID NO:29),
GGRGDGGRGDSC (SEQ ID NO:30),
GGRGDGGRGDGGRGDSC (SEQ ID NO:31),
RRRRRRRRRGDVC (SEQ ID NO:32),
CGRGDVK.amide (SEQ ID NO:33),
CGRGDV.amide (SEQ ID NO:34),
CGRGDVRGDFK.amide (SEQ ID NO:35),
CGRGDVRGDF.amide (SEQ ID NO:36),
*acetyl*-G.Apc.GDV.Apc.GDFKGGCamide (SEQ ID NO:37),
G.Apc.GDV.Apc.GDFKGGCamide (SEQ ID NO:38),
G.Apc.GDVKGGC.amide (SEQ ID NO:39),
CGGRGDS (SEQ ID NO:40),
*acetyl*-RRARGDDLDGGC.amide (SEQ ID NO:41),
CKRARGDDMDDYC (SEQ ID NO:42),
*mmp*-GGGRGDF (SEQ ID NO:43),
*acetyl*-RGDC.amide (SEQ ID NO:44),
CRGDC (SEQ ID NO:45),
CGGGRGDF (SEQ ID NO:46),
GRGDGGGGC (SEQ ID NO:47),
GRGDGGC (SEQ ID NO:48),
*ma_{Acm}*-GGGRGDF (SEQ ID NO:49),
*acetyl*-CNP.Apc.GDC (SEQ ID NO:50),
CRIARGDWNDDYC (SEQ ID NO:51),
CKFFARTVCRIARGDWNDDYCTGKSSDC (SEQ ID NO:52),
KYGGHHLGGAKQAGDV (SEQ ID NO:53),
(CH₂CO-Y_{D}.Amp.GDCKGCG.amide)₂-(ε-K)GC.amide,
or
CH₂CO-Y_{D}.Amp.GDCKGCG.amide (SEQ ID NO:54).

Each targeting peptide-containing embodiment of the invention comprises a sequence of amino acids. The term amino acid as used in this invention is intended to include all L- and D- amino acids, naturally occurring and otherwise. Single-letter abbreviations for amino acids can be found in Zubay, *Biochemistry* (2d. ed.), 1988. (MacMillen Publishing: New York) p.33; underlining indicates the formation of a thiol linkage between the linked amino acids or derivative groups; Ac=acetyl; Bz=benzoyl; Amp = 4-amidinophenylalanine; (ε-K) = lysine linked through the sidechain (epsilon) amino group; Acm=acetamidomethyl; Mob = 4-methoxybenzyl; Apc = L-S-(3-aminopropyl)cysteine; Hly=homolysine; F_{D}=D-phenylalanine; Y_{D}=D-tyrosine; ma=2-mercaptoacetic acid; mmp = 2-mercapto-2-methylpropionic acid. The list of targeting peptides set forth above is illustrative and not intended to be limiting or exclusive, and it will be understood by those with skill in the art that peptides comprising combinations of the peptides disclosed herein or their equivalents may be covalently linked to any of the thrombolytic proteinases of the invention and be within its scope.

As defined herein, a thrombolytic proteinase is "covalently linked" to a targeting compound when a covalent bond mediates the connection between the proteinase and the compound. In accordance with the invention, covalent linkage may be direct, *i.e.,* a chemical moiety on the proteinase may be linked to a chemical moiety that forms part of the targeting compound without intervening intramolecular substituents. Alternatively, covalent linkage of the thrombolytic proteinase and the targeting compound may be indirect, *i.e.,* chemical substituents may intervene between a chemical moiety on the proteinase and a chemical moiety that forms part of the targeting compound.

Targeting peptides of the present invention may be chemically synthesized *in vitro.* Such peptides may generally advantageously be prepared on a peptide synthesizer. When the targeting compounds of the invention are chemically synthesized, they may be covalently linked to the thrombolytic proteinase by a chemical conjugating moiety. In preferred embodiments, this moiety comprises a bifunctional chemical conjugating moiety. In one embodiment, the thrombolytic proteinase is conjugated with from about 1 to about 10, more preferably from about 1 to about 5 and most preferably from about 1 to about 3 copies of the targeting compound. Preferred embodiments of the chemical conjugating moieties comprising the thrombolytic agents of the invention include, but are not limited to, N-succinimidyl-2-(2-pyridyldithio) propionate, N-(γ-maleimidobutyryloxy) sulfosuccinimide ester, succinimidyl 4-(*p*-maleimidophenyl)butyrate, sulfosuccinimidyl 4-(*p*-maleimidophenyl)butyrate, 4-succinimidyloxycarbonyl-methyl-α-(2-pyridyldithio)toluene, N-hydroxysuccinimidyl-2,3-dibromopropionate, N-succinimidyl-(4-iodoacetyl)-aminobenzoate, sulfosuccinimidyl-(4-iodoacetyl)-aminobenzoate, succinimidyl-4-(N-maleimidomethyl) cyclohexane-1-carboxylate, sulfosuccinimidyl-4-(N-maleimidomethyl)cyclohexane-1-carboxylate, N-(γ-maleimidobutyryloxy) succinimide ester, *m*-maleimidobenzenyl-N-hydroxysuccinimide ester, *m*-maleimidobenzenyl-N-hydroxysulfosuccinimide ester, sulfosuccinimidyloxycarbonyl-6-(α-methyl)-α-(2-pyridyldithio) toluamide hexanoate, and sulfosuccinimidyl-6-(α-methyl-α-(2-pyridyldithio)toluamido)hexanoate.

The thrombolytic conjugates of the invention may be produced recombinantly. In this embodiment, a nucleic acid encoding one or more copies of the targeting peptide is operatively linked to a nucleic acid encoding the proteolytic enzyme to produce a conjugate-encoding nucleic acid which may be expressed in a suitable host cell to produce the thrombolytic conjugate. As used herein, "operably linked" means enzymatically or chemically ligated to form a covalent bond between the nucleic acids. Any nucleic acid may be employed to produce the thrombolytic agent of the invention, so long as the nucleic acid encodes the targeting peptide and the proteolytic enzyme and so long as the nucleic acid is capable of being expressed in a host cell. Of course, degenerate nucleic acid sequences encoding the targeting peptide and the proteolytic enzyme may be employed to produce the thrombolytic agent. Preferably, deoxyribonucleic acids are used for recombinant production of the thrombolytic agents of the invention.

In accordance with this embodiment, the thrombolytic agent-encoding nucleic acid is operably linked to an appropriate expression control sequence to form an expression vector, which is transformed (transfected) into an appropriate host cell. Any suitable expression vector may be employed to produce the thrombolytic agents of the invention recombinantly. Expression control sequences for a variety of host cells are known in the art. For mammalian expression, exemplary expression vectors include pED (Kaufman et al. (1991) Nucleic Acids Res. **19,** 4484-4490; pEF-BOS (Mizushima et al. (1990) Nucleic Acids Res. **18,** 5322; pXM, pJL3 and pJL4 (Gough et al. (1985) EMBO J. **4,** 645-653; pMT2 (derived from pMT2-VWF, A.T.C.C. # 67122, see PCT/US87/00033); and the like. Suitable expression vectors for use in yeast and bacterial cells are also known, and expression vectors for insect cells are set forth, for example, in U.S. Pat.No. 5,004,687 and in Summers and Smith, *Texas Agricultural Experiment Station Bulletin No. 1555* (1987). Construction of such expression vectors is well within the level of skill in the art.

Suitable host cells include, for example, mammalian cells such as monkey COS cells, Chinese hamster ovary (CHO) cells, human kidney 293 cells, human epidermal A431 cells, human Colo205 cells, mouse 3T3 cells, CV-1 cells, other transformed primate cell lines, normal diploid cells, cell strains derived from *in vitro* culture of primary tissue, primary explants, HeLa cells, mouse L cells, BHK cells, HL-60 cells, U937 cells, HaK cells, and the like. Materials and methods for baculovirus/insect cell expression are commercially available, *e.g.*, from Invitrogen, San Diego, California, USA (the MaxBac^{®} kit). Suitable yeast strains include, for example, *Saccharomyces cerevisiae, Schizosaccharomyces pombe, Kluyveromyces* strains, *Candida,* or any yeast strain capable of expressing heterologous proteins. Suitable bacterial strains include *Escherichia coli, Bacillus subtilis,* attenuated strains of *Salmonella typhimurium,* or any bacterial strain capable of expressing heterologous proteins.

The thrombolytic agents of the invention may also be expressed as a product of transgenic animals, *e.g.*, as a component of the milk of transgenic cows, goats, pigs, or sheep which are characterized by somatic or germ cells containing a nucleic acid sequence encoding the conjugates.

In certain embodiments of the thrombolytic agents of this invention, the agent is radiolabeled, preferably with a gamma-emitting radioisotope such as Tc-99m, ¹¹¹In, ⁶⁸Ga, ¹²³I or ¹³¹I (*see, for example,* Pearson *et al.,* 1996, *J. Med. Chem.* 39: 1372-1382). Radioactively-labeled thrombolytic agents provided by the present invention are provided having a suitable amount of radioactivity. Generally, the dose of radiolabeled thrombolytic agent administered has a radioactivity of about 0.01 mCi to about 100 mCi, preferably 1 mCi to 20 mCi.

An advantageous feature of the thrombolytic agents of the invention is that localization of such agents can be visualized, and the course of thrombolysis can be monitored, *in vivo,* and in real time. Administration of radiolabeled thrombolytic agents can therefore be used to closely monitor course of treatment and facilitate choice and administration of therapeutically effective amounts of the thrombolytic agents of the invention.

A low dosage of a labeled form of the thrombolytic agent of the invention may also be used to image thrombi for diagnostic purposes. In this embodiment, any signal-generating label may be used. For example, radiolabels, fluorescent labels, paramagnetic labels, heavy elements or rare earth ions suitable for use in computerized tomography, and the like, may be used to label the thrombolytic agent of the invention for diagnostic purposes.

As used herein, the term "therapeutically effective amount" means the total amount of each active component of the pharmaceutical composition containing the thrombolytic agent of the invention or method employing the thrombolytic agent of the invention which is sufficient to show a meaningful patient benefit, *i.e*., reduction in the incidence and severity of thrombi as compared to that expected for a comparable group of patients not receiving the thrombolytic agent of the invention, as determined by the attending physician. When applied to an individual active ingredient administered alone, the term refers to that ingredient alone. When applied to a combination, the term refers to combined amounts of the active ingredients that result in the therapeutic effect, whether administered in combination, serially, or simultaneously.

The thrombolytic agents provided by the present invention are provided as pharmaceutical compositions for lysing thrombi *in vivo.* In accordance with the teachings of this invention, the thrombolytic agents are preferably administered in a single unit injectable dose. These agents can be administered intravenously in any conventional medium for intravenous injection such as an aqueous saline medium, or in blood plasma medium. The solution to be injected at unit dosage is from about 0.01mL to about 10mL. The thrombolytic agents of the invention are preferably administered at a dose of from about 0.1 to about 10 mg/kg body weight, administered intravenously either totally as a bolus or partly as a bolus followed by infusion over 1-2 hours. In radiolabeled embodiments of the thrombolytic agents, the unit dose to be administered has a radioactivity of about 0.01 mCi to about 100 mCi, preferably 1 mCi to 20 mCi. After intravenous administration, the thrombus site is monitored, in certain embodiments by radioimaging *in vivo.*

The methods for making, radiolabeling and using the thrombolytic agents of the invention are more fully illustrated in the following examples, which are are shown by way of illustration and not by way of limitation.

### EXAMPLE 1

### Purification of Fibrolase

Fibrolase was purified from *Agkistrodon contortrix contortrix* venom using a biochemical purification protocol as disclosed in Loayza *et al.* (1994, *J. Chromatog. B* 662: 227-243).

Briefly, crude snake venom was obtained from Biotoxins, Inc. (St. Cloud, FL) or from the Miami Serpentarium Laboratory (Punta Gorda, FL). All purification steps were performed at 4°C. Ten grams of venom were applied in five applications to a 250 mm hydrophobic interaction chromatography column (HIC; Poly Propyl A, Poly LC, Western Analytical Products, Temecula, CA) having a 21 mm internal diameter. Venom was dissolved at a concentration of 2 g per 9 mL HIC buffer A (comprising 0.1M phosphate, 1M ammonium sulfate, and 0.02% sodium azide, pH 6.8) and subjected to centrifugation at 9000*g* for 30 minutes at 4°C to remove particulate contaminants. The resulting supernatant solution was filtered through a 0.2 µm membrane and then applied to the HIC column. Venom proteins were eluted from the column using a gradient elution protocol as follows: (a) 50 minutes isocratically at 100% HIC buffer A; (b) 90 minutes using a linear gradient to 100% HIC buffer B (comprising 0.1M phosphate and 0.02% sodium azide, pH 6.8); and (c) 60 minutes isocratically at 100% HIC buffer B. Flow rate through the column was maintained at 5 mL/minute, and 5 mL fractions were collected. Fractions were monitored spectrophotometrically using absorbance at 280 nm, and analyzed for fibrinolytic activity, and by sodium dodecyl sulfate-polyacrylamide gel electrophoresis (SDS-PAGE).

Fractions containing fibrinolytic activity were pooled, concentrated using 80% ammonium sulfate, and dialyzed against hydroxyapatite (HAP) buffer (comprising 0.01M phosphate, 0.02% sodium azide, pH 6.8). This dialyzed sample was concentrated using either a stirred cell containing a YM 10 membrane or a Centricon-10 concentrator (both obtained from Amicon, Beverly, MA) to a final volume of about 10 mL.

This concentrated sample was then applied to a 100 mm hydroxyapatite (HAP) column (SynChropak HAP-5, SynChrom, Lafayette, IN) having a 21.1 mm internal diameter. Venom proteins were eluted from this column using a gradient elution protocol as follows: (a) 15 minutes isocratically at 100% HAP buffer A; (b) 130 minutes using a linear gradient to 60% HAP buffer B (comprising 0.35M phosphate and 0.02% sodium azide, pH 6.8); and (c) 35 minutes linear gradient to 100% HAP buffer B. Flow rate through the column was maintained at 2 mL/minute, and 2 mL fractions were collected. As above, fractions were monitored spectrophotometrically using absorbance at 280 nm, and analyzed for fibrinolytic activity.

Anion-exchange chromatography using a 100 mm high resolution fast protein liquid chromatography column (Mono Q, Pharmacia LKB, Piscataway, NJ) having an internal diameter of 16 mm was used for removal of minor contaminants from fractions containing fibrinolytic activity. HAP chromatography fractions having fibrinolytic activity were pooled, concentrated using the Amicon stirred cell as above, and dialyzed against several changes of Mono Q buffer A (comprising 20 mM Tris-HCl, pH 8) overnight. A volume corresponding to about 18mg protein was filtered through a 0.2 µm membrane and applied to the Mono Q column. Venom proteins were eluted from this column using a gradient elution protocol as follows: (a) 10 minutes isocratically at 100% Mono Q buffer A; (b) 90 minutes using a linear gradient to 20% Mono Q buffer B (comprising 20mM Tris-HCl, 500mM NaCl, pH 8); (c) 5 minutes linear gradient to 100% Mono Q buffer B; and (d) 5 minutes isocratically at 100% Mono Q buffer B. Flow rate through the column was maintained at 4 mL/minute, and 2 mL fractions were collected. As above, fractions were monitored spectrophotometrically using absorbance at 280 nm, and analyzed for fibrinolytic activity and by SDS-PAGE.

This purification protocol resulted in a fibrolase preparation purified from other proteins originally present in snake venom. SDS-PAGE analysis of Mono Q column fractions having fibrinolytic activity showed purification to homogeneity, comprising a single band having an apparent molecular weight of about 23kD. Further analysis, however, revealed that native fibrolase purified using this scheme consisted of two isoforms, detectable using extremely narrow range (pH 6.6-6.9) isoelectric focusing gel electrophoresis. The two isoforms of fibrolase were separated using a weak cationic exchange HPLC protocol, as follows. Approximately 9mg of protein from the Mono Q fraction pool was dialyzed against CM buffer A (comprising 30mM 2-(N-morpholino)-ethansulfonic acid, sodium salt (NaMES), pH 6.4) and concentrated using the Amicon stirred cell. The sample was filtered through a 0.2 µm membrane and applied to a 250 mm carboxymethyl cellulose HPLC column (SynChropak CM 300, SynChrom; 10mm internal diameter). Fibrolase isoform proteins were eluted from this column using a gradient elution protocol as follows: (a) 10 minutes isocratically at 100% CM 300 buffer A; (b) 95 minutes using a linear gradient to 30% CM 300 buffer B (comprising 30mM NaMES, 300mM NaCl, pH 6.4); (c) 40 minutes linear gradient to 100% CM 300 buffer B; and (d) 10 minutes isocratically at 100% CM 300 buffer B. Flow rate through the column was maintained at 2.4 mL/minute, and 1.2 mL fractions were collected.

To completely resolve the isoforms, pooled fractions containing each of the isoforms fib1 and fib2 were dialyzed against CM 300 buffer A, reapplied to the column and eluted as described. Fractions containing fibrinolytic activity arising from each of the fibrolase isoforms were pooled, concentrated and exchanged to a buffer comprising 20 mM HEPES, 50 mM NaCl, pH 7.2, diluted (or concentrated) to a concentration of about 1mg/mL, and stored at -80° until use. Protein concentration was determined using a commerciallyavailable assay (BCA, Pierce Chemical Co., Rockford, IL).

Alternatively, recombinant fibrolase can be produced using the methods of European Patent Application EP 323722 and Loayza *et al.* (*Ibid.*), the teachings of these references being incorporated by reference herein.

### EXAMPLE 2

### Solid Phase Peptide Synthesis

Solid phase peptide synthesis (SPPS) was carried out on a 0.25 millimole (mmole) scale using an Applied Biosystems Model 431A Peptide Synthesizer and using 9-fluorenylmethyloxycarbonyl (Fmoc) amino-terminus protection, coupling with dicyclohexylcarbodiimide/hydroxybenzotriazole or 2-(1H-benzotriazol-1-yl)-1,1,3,3-tetramethyluronium hexafluorophosphate/ hydroxybenzotriazole (HBTU/HOBT), and using *p*-hydroxymethylphenoxy-methylpolystyrene (HMP) resin for carboxyl-terminus acids or Rink amide resin for carboxyl-terminus amides. Resin-bound products were routinely cleaved using a solution comprised of trifluoroacetic acid, water, thioanisole (if an arginine residue is contained in the peptide), ethanedithiol, and triethylsilane, prepared in ratios of 100 : 5 : 5 : 2.5 : 2 for 0.5 - 3 hours at room temperature.

Where appropriate, N-terminal acetyl groups were introduced by treating the free N-terminal amino peptide bound to the resin with 20% v/v acetic anhydride in NMP (N-methylpyrrolidinone) for 30 minutes. Where appropriate, 2-chloroacetyl and 2-bromoacetyl groups were introduced either by using the appropriate 2-halo-acetic acid as the last residue to be coupled during SPPS or by treating the N-terminus free amino peptide bound to the resin with either the 2-halo-acetic acid/ diisopropylcarbodiimide/ N-hydroxysuccinimide in NMP or the 2-halo-acetic anhydride/ diisopropylethylamine in NMP. Where appropriate, 2-haloacetylated peptides were cyclized by stirring an 0.1 - 1.0 mg/mL solution in phosphate or bicarbonate buffer (pH 8) containing 0.5 - 1.0 mM EDTA for 4 - 48 hours, followed by acidification with acetic acid, lyophilization and HPLC purification. Where appropriate, Cys-Cys disulfide bond cyclizations were performed by treating the precursor cysteine-free thiol peptides at 0.1 mg/mL in pH 7 buffer with aliquots of 0.006M K₃Fe(CN)₆ until a stable yellow color persisted. The excess oxidant was reduced with excess cysteine, the mixture lyophilized and then purified by HPLC.

Where appropriate, the amino acids 4-amidinophenylalanine and L-(S-3aminopropyl) cysteine are prepared using the protocol of Pearson *et al.,* 1996, *J. Med. Chem.* 39: 1372-1382, incorporated by reference herein.

Crude peptides were purified by preparative high pressure liquid chromatography (HPLC) using a Waters Delta Pak C 18 column and gradient elution using 0.1% trifluoroacetic acid (TFA) in water modified with acetonitrile. Acetonitrile was evaporated from the eluted fractions which were then lyophilized. The identity of each product was confirmed by fast atom bombardment mass spectroscopy (FABMS) or electrospray mass spectrometry (ESMS).

### EXAMPLE 3

### Crosslinking Fibrolase with Chemical Crosslinking Agents

### 1. Crosslinking fibrolase with S-GMBS via a primary amine

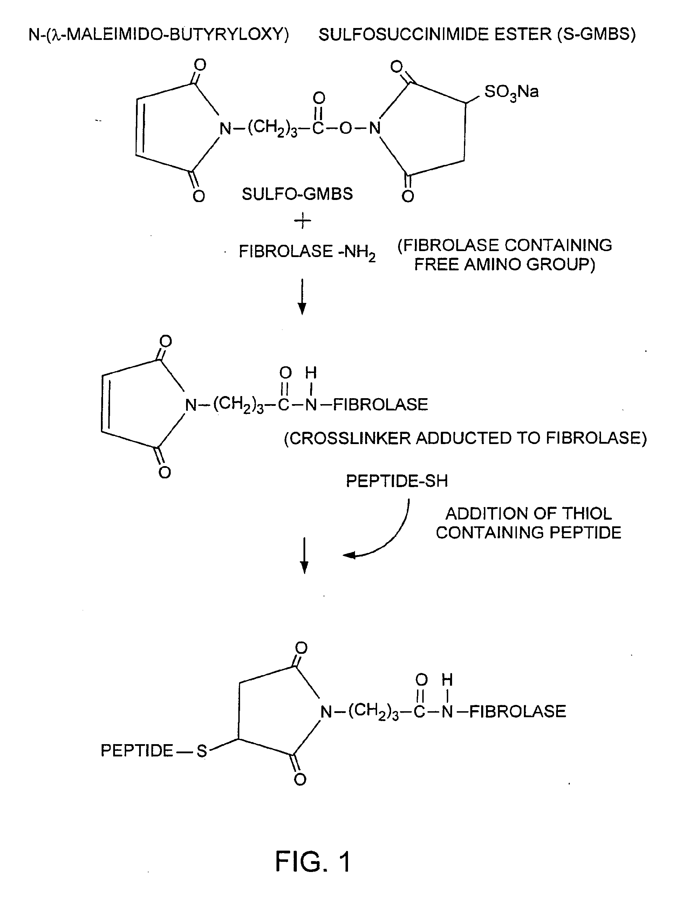

Fibrolase isoform 2 (fib2) was prepared as described in Example 1. N-(γ-maleimidobutyryloxy) sulfosuccinimide ester (S-GMBS, shown in Figure 1 and obtained from Pierce) was dissolved in water to a final concentration of 25mM. Fibrolase and S-GMBS were combined at a 1:20 molar ratio in a total volume of ImL, and allowed to react for 30 minutes at room temperature. After this reaction time, excess S-GMBS was removed using an Amicon Centricon-10 centrifugal concentrator. The reaction was diluted with an additional 1 mL of fibrolase dilution buffer, applied to the concentrator and centrifuged until the volume was reduced to about 200 µL. An additional 1.8 mL buffer was added and the volume again reduced to 200 µL by centrifugation. This protocol was repeated until a volume of about 10 mL had been passed through the concentrator, corresponding to about a 1:1000 dilution of the reagents comprising the fibrolase:S-GMBS reaction mixture. The final retentate from the concentrator was diluted with buffer to a final volume of 1 mL, and the fibrolase concentration determined to be 0.84 mg/mL using the BCA reagent and protocol (Pierce). Coupling reactions (described below) were performed within one hour after chemical crosslinking.

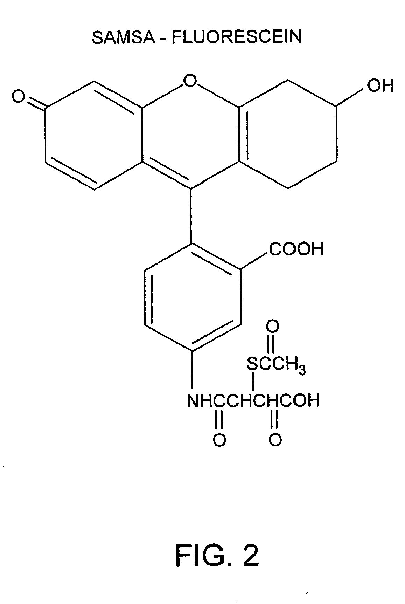

The extent of S-GMBS adduction to fibrolase was determined using a fluorescent detection reagent (SAMSA fluorescein, Figure 2, obtained from Molecular Probes, Eugene OR). This reagent (after strong base activation) forms a covalent linkage with thiol-reactive groups on protein modification reagents such as S-GMBS. The absorbance and fluorescent properties of SAMSA-fluorescein have been extensively characterized, which permits quantitation of the number of SAMSA-fluorescein adducts formed per fibrolase molecule, which in turn provides an accurate estimate of the average number of crosslinking agents that have been covalently linked to each fibrolase molecule. (Fibrolase contains no free sulfhydryl groups that could react with SAMSA-fluorescein.)

SAMSA-fluorescein was activated by incubation of 1mg of the reagent in 100 µL 0.1M NaOH for 15 minutes at room temperature. The solution was then neutralized by the addition of 14 µL of 6M HCl buffered with 200 µL 0.5M sodium phosphate, pH 7. An aliquot of S-GMBS crosslinked fibrolase was then combined with activated SAMSA-fluorescein at a 1:10 molar ratio and incubated at room temperature for 30 minutes. Unbound SAMSA-fluorescein was removed as described above using a Centricon concentrator unit having a 3000 dalton molecular weight cut-off; ten volumes of buffer were exchanged with the SAMSA-fluorescein treated fibrolase reaction mixture. After concentration, the absorbance of crosslinked protein-SAMSA fluorescein was determined at 495 nm. Based on an extinction coefficient of 80,000 cm⁻¹M⁻¹, the number of free S-reactive maleimide groups from S-GMBS covalently linked to fibrolase was calculated to be 1.7 per molecule of fibrolase. This assay also demonstrates that there were 1.7 free maleimide groups per fibrolase molecule available for reaction with peptide (as described below). Control reactions were performed using buffer containing no fibrolase, as well as buffer containing uncrosslinked fibrolase, each of which provided a determination of non-specific background fluorescence.

### 2. Crosslinking fibrolase with SPDP and S-SMPT

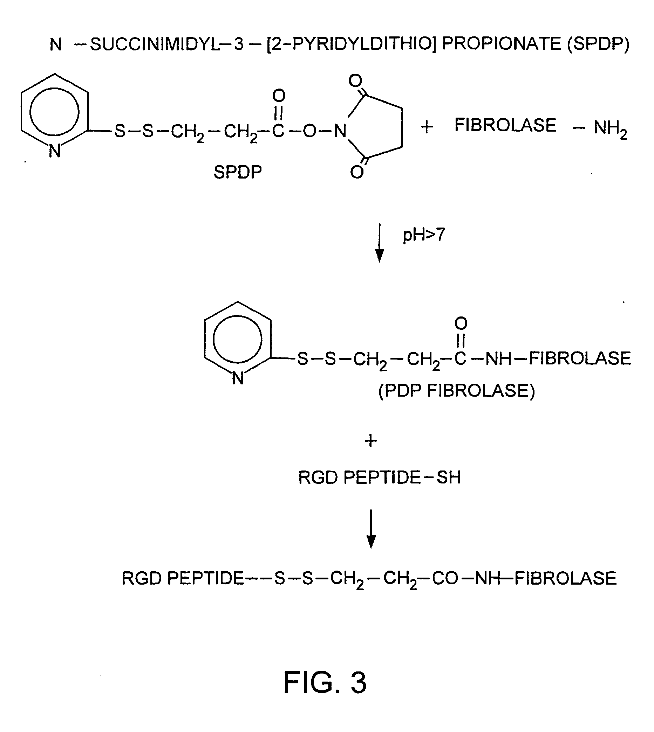

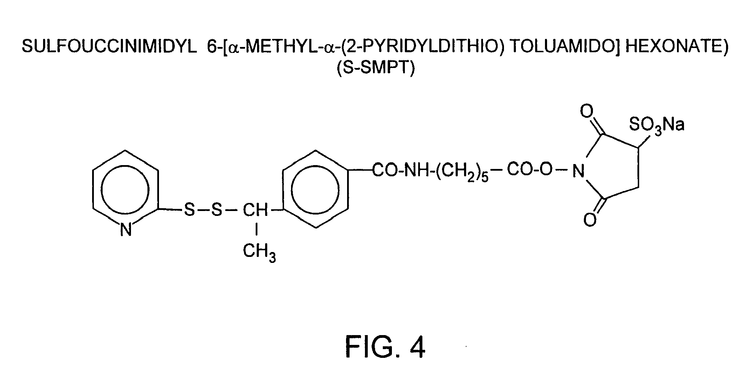

Fibrolase isoform 2 (fib2) was prepared as described in Example 1. A solution of fibrolase at a concentration of 2.6 mg/1.2 mL in 50mM HEPES, 100 mM NaCl, pH 7.5 was treated with a 10-fold molar excess of N-succinimidyl-3-(2-pyridyldithio) propionate (SPDP, Figure 3) or sulfosuccinimidyl-6-(α-methyl-α-(2-pyridyldithio) toluamido) hexonoate (S-SMPT, Figure 4). (Stock solutions of each of these reagents were prepared at a concentration of 20 mM in dimethylsulfoxide.) Each mixture of reagent and fibrolase was stirred gently at room temperature for 30 minutes. Excess crosslinking reagent was removed by gel filtration using a Sephadex G-25 column or by centrifugation using the Centricon-10 concentrator as described above. Final protein concentration was determined using the BCA reagents and protocol.

Crosslinking efficiency of both SPDP and S-SMTP was determined by the production of pyridine-2-thione from crosslinked fibrolase after treatment with dithiothreitol. An aliquot of crosslinked fibrolase (220 µg in 1.2 mL) was treated with 0.1 mL 25 mM dithiothreitol at room temperature for 30 minutes. The amount of pyridine-2-thione released from the SPDP and S-SMTP crosslinking agents was detected spectrophotometrically at 343 nm, and the corresponding amount of each crosslinker covalently linked to fibrolase calculated using the extinction coefficient of pyridine-2-thione, 80,800 cm⁻¹M⁻¹. Using this assay, the crosslinking efficiencies of both SPDP and S-SMTP were found to be nearly identical, there being 1.7 mol of either crosslinker per mol fibrolase protein.

### EXAMPLE 4

### Coupling Crosslinked Fibrolase with Peptide

### 1. Coupling peptide with S-GMBS crosslinked fibrolase

A peptide having the structure CH₂CO-Y_{D}.Amp.GDCKGCG.amide (SEQ ID NO:54, abbreviations and other conventions are as disclosed above) was coupled to S-GMBS crosslinked fibrolase as follows. The peptide was added to crosslinked fibrolase at a molar ratio of 5:1 peptide:crosslink ends (equivalent to a molar ratio of 5:1.7 peptide: fibrolase for the crosslinked fibrolase preparations of Example 3). Lyophilized peptide was slowly added to a stirred fibrolase solution to permit peptide to slowly and completely dissolve. Reaction was performed under a gentle stream of nitrogen for an initial 30 minutes, and then the reaction mixture was capped and incubated at room temperature for 15 hours. After this incubation, excess unconjugated peptide was removed from the conjugated fibrolase using a centrifugal concentrator having a 10,000 dalton molecular weight cutoff. An aliquot of the final conjugate was tested with SAMSA-fluorescein to determine the extent of unconjugated crosslinked fibrolase. This assay determined that an average of 1.7 mol peptide were adducted per mol fibrolase, indicating that essentially all of the free maleimide reactive groups on the crosslinked fibrolase had been conjugated with peptide.

### 2. Coupling peptide with SPDP- and S-SMPT-crosslinked fibrolase

A peptide having the structure CH₂CO-Y_{D}.Amp.GDCKGCG.amide (SEQ ID NO:54) was coupled to SPDP and S-SMTP crosslinked fibrolase as follows. The peptide was added to crosslinked fibrolase at a molar ratio of 2:1 peptide:crosslink ends (equivalent to a molar ratio of 2:1.7 peptide: fibrolase for the crosslinked fibrolase preparations of Example 3). Lyophilized peptide was slowly added to a stirred fibrolase solution to permit peptide to slowly and completely dissolve. Reaction was performed under a gentle stream of nitrogen for an initial 5 minutes, then the reaction mixture was capped and incubated at room temperature overnight. After this incubation, excess unconjugated peptide was removed from the conjugated fibrolase using a centrifugal concentrator having a 10,000 dalton molecular weight cutoff. An aliquot of the final conjugate was tested with SAMSA-fluorescein to determine the extent of unconjugated crosslinked fibrolase. This assay determined that an average of 1.7 mol peptide were adducted per mol fibrolase, indicating that essentially all of the free maleimide reactive groups on the crosslinked fibrolase had been conjugated with peptide.

### 3. HPLC purification of fibrolase:peptide conjugates

Peptide:fibrolase conjugates were purified by HPLC as follows. For example, SPDP crosslinked conjugates were applied to a 200mm cation exchange column (Poly Cat A, Western Analytical Products, Inc., Temecula, CA) in NaMES buffer at a flow rate of 1mL/minute. Conjugates were eluted from the column using an elution gradient of 0 to 30% elution buffer B (comprising 30 mM NaMES and 0.3M NaCl, pH 6.4) over 95 minutes, followed by 40 minutes elution with 100% elution buffer B.

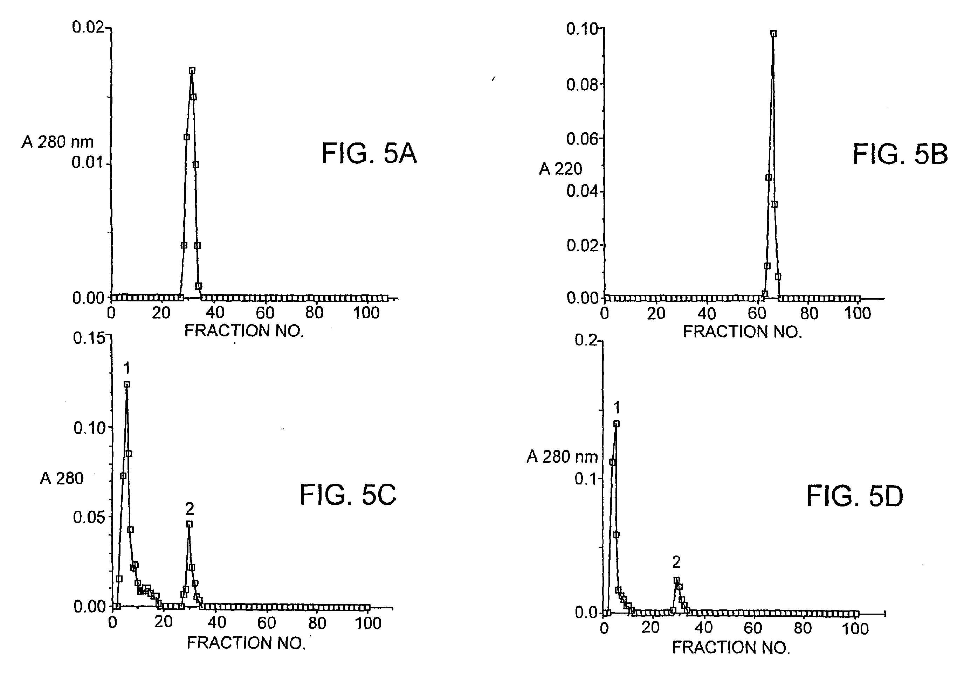

Results of this purification protocol are shown in Figure 5. Panel A illustrates the elution profile of unconjugated fibrolase isoform 2 control, which eluted from the column at 29.9 minutes, and Panel B shows the elution profile of unconjugated peptide, which eluted from the column at 66.7 minutes. The elution profile of fibrolase crosslinked to SPDP showed two peaks: a first peak at 6.8 minutes representing PDP-fibrolase, and a second peak at 29 minutes corresponding to unmodified fibrolase (Panel C). Peptide-fibrolase conjugates eluted from the column as shown in Panel D at 6.3 minutes, while unconjugated fibrolase eluted at 29 minutes as previously described. The amount of peptide:fibrolase conjugate obtained using this protocol was 1.46 mg from a starting amount of 2.6 mg fibrolase, an overall yield of 56.5%.

The chemical identity of the conjugate was determined by amino acid composition analysis. Conjugate samples (33-50 mg) were hydrolyzed in 6N HCl for 90 minutes at 150°C. The hydrolysates were derivatized with phenylisothiocyanate and analyzed using an automated amino acid analyzer (Model 420A, obtained from Applied Biosystems, Inc., Foster City, CA). Analysis was performed in triplicate on a 2.1 x 220mm PTC-18 HPLC column at a flow rate of 300 mL/minute using an ultraviolet detector set at 254 nm. Amino acid composition analyses were performed in triplicate.

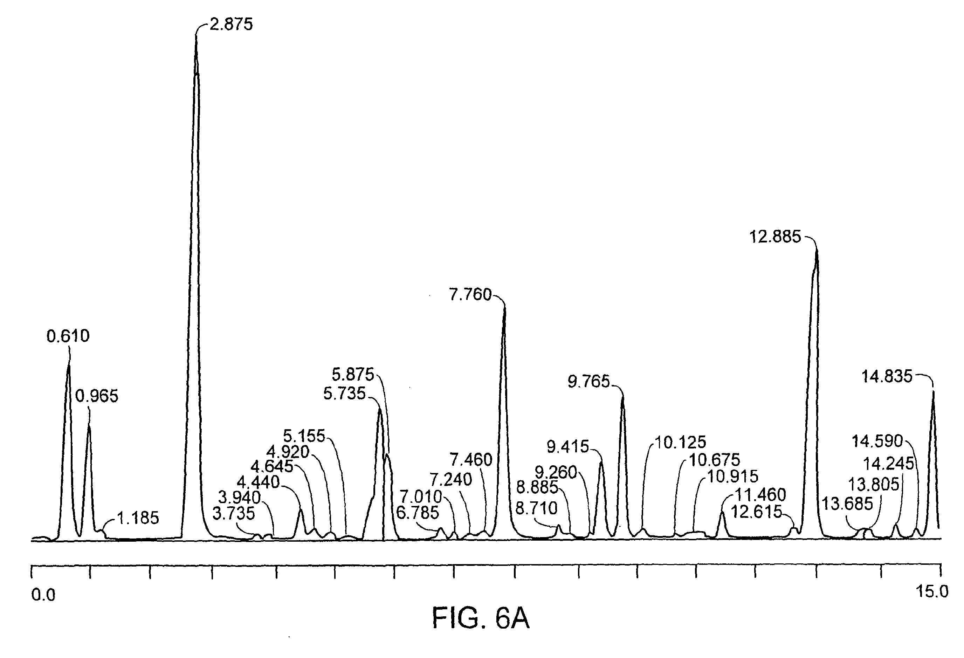

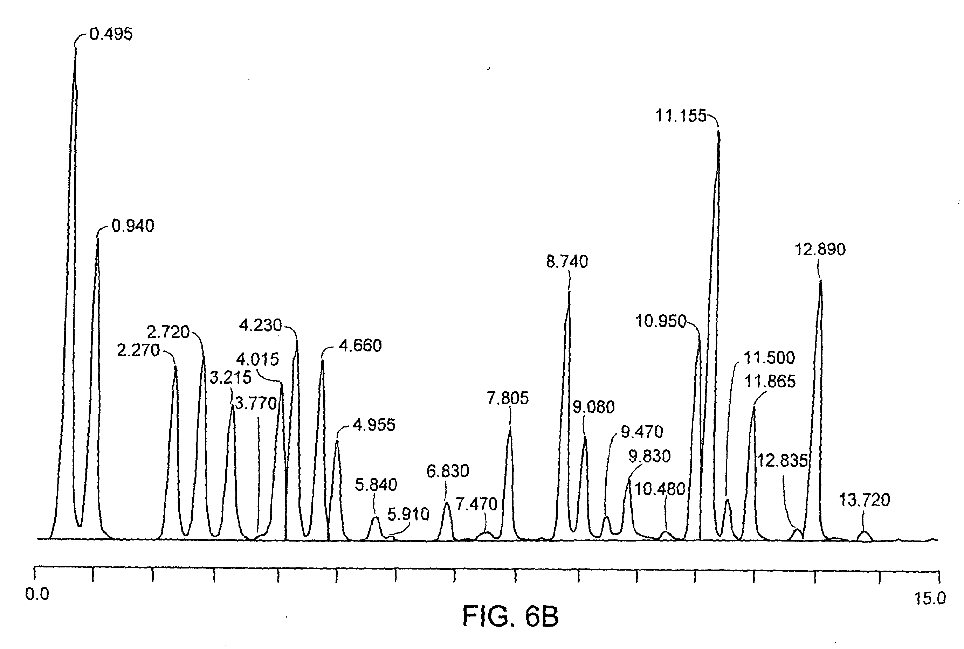

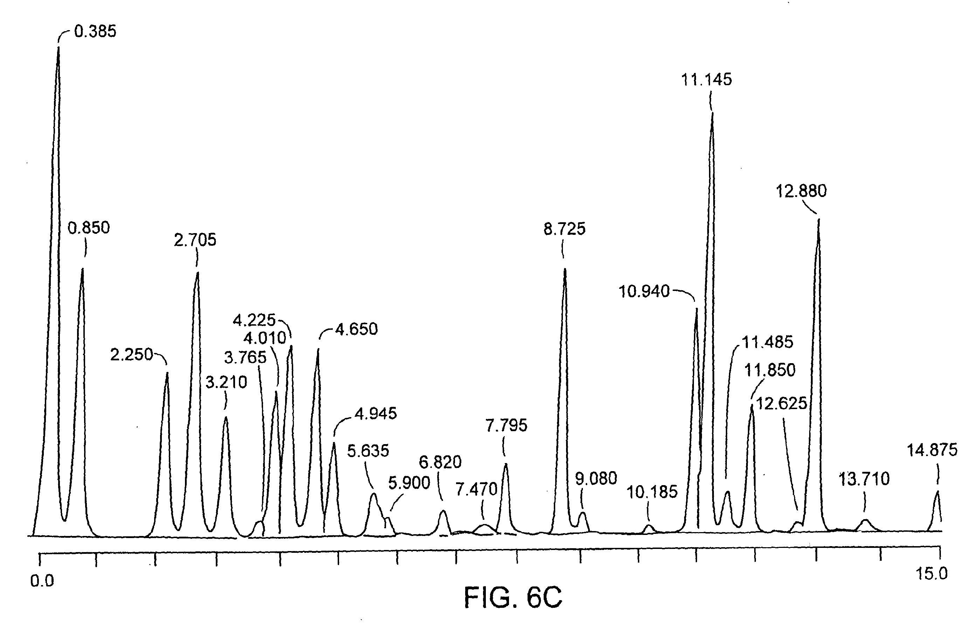

The results of these analyses are shown in Table I and in Figure 6. Fibrolase is composed of 203 amino acids (as described in Randolph *et al.,* 1992, *Prot. Sci.* 1: 590-600), and the peptide is made up of 9 amino acids. A unique arginine derivative (shown as peak 14.8 minutes in Figure 6) is derived from the peptide; observation of this peak in the purified conjugate confirmed peptide:fibrolase conjugation at a molar ratio of from about 1:1 to about 2:1.

**TABLE I**

| Amino Acid | Fibrolase | | Conjugate | | Peptide | |
|---|---|---|---|---|---|---|
| | Obs. | Exp. | Obs. | Exp. | Obs. | Exp. |
| | | | | | | |
| Asx | 31 | 31 | 37 | 37 | | 1 |
| Glx | 20 | 20 | 18 | 18 | 1 | |
| Ser | 12 | 12 | 14 | 14 | | |
| Gly | 12 | 12 | 15 | 15 | 3 | 3 |
| His | 10 | 10 | 10 | 10 | | |
| Arg | 1 | 9 | 9 | 9 | | |
| Thr | 13 | 13 | 16 | 16 | | |
| Ala | 10 | 11 | 10 | 10 | | |
| Pro | 6 | 5 | 5 | 5 | | |
| Tyr | 5 | 5 | 5 | 5 | 1 | 1 |
| Val | 11 | 14 | 12 | 12 | | |
| Met | 7 | 6 | 6 | 6 | | |
| Cys | 4 | 6 | 6 | 6 | 1 | 2 |
| Ile | 9 | 12 | 9 | 9 | | |
| Leu | 19 | 21 | 1 | 18 | | |
| Phe | 6 | 6 | 6 | 6 | | |
| Lys | 7 | 7 | 8 | 8 | 1 | 1 |
| Trp | ND | 3 | ND | 3 | | |
| Arg Deriv. | - | - | - | - | 1 | 1 |
| Total | | 203 | | 208 | | 9 |

### EXAMPLE 5

### Proteolytic Activity of Peptide:Fibrolase Conjugates

The proteolytic activity of peptide:fibrolase conjugates was determined using two substrates: azocasein (to determine non-specific protease activity) and fibrin (to determine fibrinolytic activity and to compare this activity between purified fibrolase and the peptide:fibrolase conjugate). These assays were performed as follows.

Azocasein assays were performed as described in Retzios *et al.* (1990, *Prot. Express. Purific.* 1: 33-39). Briefly, azocasein was prepared by dissolving 2.5 g azocasein (Cal BioChem, San Diego, CA) in 50 mL of a 1% solution of NaHCO₃. This solution was gradually heated to 60°C with stirring until the protein was totally dissolved. This solution was dialyzed overnight against 4 L of a 1% NaHCO₃ solution, and the protein precipitated by the addition of an equal volume of 10% trichloroacetic acid (TCA). Protein was collected by centrifugation, the supernatant discarded and the protein pellet resuspended in 50 mL of a 1% solution of NaHCO₃. This solution was again dialyzed overnight against 4 L of a 1% NaHCO₃ solution.

In performing the assay, 1 mL of the above azocasein reagent solution was combined with 50 µL of the sample and incubated at 37°C for 30 minutes. After this incubation, 1 mL of a 1.16M perchloric acid solution was added. The reaction mixture was centrifuged on a tabletop centrifuge for 10 minutes, and absorbance at 390-440 nm was determined against the appropriate sample blank. Proteolytic activity was calculated using a standard curve of activity *versus* absorbance.

Fibrinolytic activity was detected using a fibrin plate assay described in Bajwa *et al.* (1980, *Toxicon* 18: 285-289). Briefly, a fibrin plate was prepared by pipetting a fibrinogen solution (6 mL 1.65% human fibrinogen in 0.07M ammonium sulfate, 50mM sodium barbital, 93mM NaCl, 1.66mM CaCl₂, and 0.69mM MgCl₂, pH 7.5) onto a 90 x 15 mm petri dish after mixing with 0.2 mL of a topical thrombin solution (20 NIH units/mL in 150 mM NaCl, pH 7.5; obtained from Parke-Davis, Detroit, MI) containing 0.25% gelatin (Difco labs, Detroit, MI). Plates were allowed to stand at room temperature for at least 30 minutes on a level surface to form the fibrin layer. Small (2 mm diameter) holes comprising sample wells were made in the fibrin layer, spaced at 1.5 cm intervals on the plate. Samples were added to the sample wells in 10 µL, and the plates incubated at 37°C for 18 hours. Fibrin plate clearance was determined as the increase in diameter of the sample well due to fibrinolytic activity of the sample. Alternatively, a 24-well plate was used to perform this assay, wherein each well contains 300 µL of a fibronogen solution, into which is added 10 mL of a thrombin solution to initiate fibrin formation.

The results of these assays are shown in Table II. The proteolytic activities of fibrolase, PDP-fibrolase and peptide:fibrolase conjugate are shown. The SPDP modification of two ε-amino groups in fibrolase did not appear to have any significant effect on fibrolase activity in either assay. The derivatized fibrolase in each instance retained over 90% of the proteolytic activity of native fibrolase. Subsequent conjugation of PDP-fibrolase with the peptide (SEQ ID NO:54) also produced no dramatic change in proteolytic activity, the conjugate displaying 79-82% of native fibrolase activity in both assays. Results similar to these results (obtained with a crosslinking agent having a 6.8 A spacer carbon chain) were obtained with S-SMPT crosslinked conjugates (wherein the SMPT moiety has a 20 A spacer arm) and with S-GMBS crosslinked conjugates (which showed no reduction in proteolytic activity in either assay). These results demonstrate that derivatization of fibrolase isoform 2 with a variety of chemical crosslinking agents and that conjugation with a platelet-specific peptide does not adversely affect the proteolytic, and more importantly, the fibrinolytic activity of fibrolase.

**TABLE II**

| Enzyme | Specific Activity | | | |
|---|---|---|---|---|
| | Azocasein (U/mg) | | Human Fibrin (U/mg) | |
| | | %^{a} | | %^{a} |
| Fibrolase | 1.22 ± 0.05 | 100 | 32.2 ± 2.5 | 100 |
| PDP fibrolase | 1.12 ± 0.03 | 92 | 29.6 ± 2.5 | 92 |
| PDP conjugate | 1.00 ±0.05 | 82 | 26.4 ± 2.5 | 82 |
| S-SMPT conjugate | 0.98 ± 0.05 | 80 | 27.0 ± 2.5 | 84 |
| S-GMBS conjugate | 1.20 ± 0.05 | 98 | 35.1 ± 1.8 | (100) |

| | | | | |
|---|---|---|---|---|
| One azocasein unit was defined as the change in absorbance at 390 nm/minute One fibrinolysis unit was defined as the area of lysis of 1.0µg in mm² a = percentage of fibrolase activity | | | | |

### EXAMPLE 6

### Platelet Aggregation Inhibition Assays of Peptide:Fibrolase Conjugates

Platelet aggregation studies were performed essentially as described by Zucker (1989, *Methods in Enzymol.* 169: 117-133). Briefly, platelet aggregation was assayed with or without putative platelet aggregation inhibitory compounds using fresh human platelet-rich plasma (PRP), comprising 250,000 platelets per µL. Whole human blood (36 mL) was freshly drawn from volunteers who were medication-free for at least 2 weeks prior to blood draw. Blood was drawn into 4mL of a 0.1M sodium citrate solution and centrifuged (150g for 20 minutes at 22°C) to pellet red blood cells. The supernatant, comprising PRP, was removed and the remaining blood re-centrifuged at 8000g for 10 minutes to produce platelet-poor plasma (PPP; used to dilute the PRP to a final concentration of 250,000 platelets/µL). Alternatively, PPP was prepared by centrifugation of PRP for 1 minute in a tabletop microcentrifuge. PPP is also used as a negative control for platelet aggregation.

Platelet aggregation was induced by the addition at 37°C of a solution of adenosine diphosphate (ADP) to a final concentration of 20 µM, and the extent of platelet aggregation monitored using a four channel aggregometer (Model IV Plus, Helena Laboratories, Beaumont, TX). The concentrations of platelet aggregation inhibitory compounds used were varied from 0.1 to 500 µg/mL, and these compounds were added to PRP immediately prior to (about 1 minute before) addition of the ADP solution used to induce platelet aggregation. The concentration of inhibitor that reduced the extent of platelet aggregation by 50% (defined as the IC₅₀) was determined from plots of inhibitor concentration *versus* extent of platelet aggregation. Inhibition curves for a variety of disintegrins comprising the peptide sequence RGD was determined for each batch of platelets tested as positive controls.

The results of these experiments are shown in Table III. Native fibrolase was determined to have an IC₅₀ value of greater than 1300 nM, while the peptide (SEQ ID NO:54) showed an IC₅₀ value of 67nM. The peptide (SEQ ID NO:54) conjugated with S-SMPT was determined to have an IC₅₀ of 300 nM, while the S-GMBS crosslinked embodiment showed an IC₅₀ of 97 nM. These results illustrate that the peptide:fibrolase conjugates of the invention retain the capacity to inhibit platelet aggregation *in vitro,* a property strongly correlated with platelet binding and antithrombotic activity *in vivo.*

**TABLE III**

| Compound | IC₅₀(nM) |
|---|---|
| | |
| Fibrolase | >1600 |
| Peptide (SEQ ID NO:54) | 67 |
| Peptide (SEQ ID NO:54)-SPDP-fibrolase conjugate | 300 |
| Peptide (SEQ ID NO:54)-S-SMPT-fibrolase conjugate | 300 |
| Peptide (SEQ ID NO:54)-S-GMBS-fibrolase conjugate | 97 |

It should be understood that the foregoing disclosure emphasizes certain specific embodiments of the invention and that all modifications or alternatives equivalent thereto are within the scope of the invention as set forth in the appended claims.

## Claims

1. A thrombolytic agent comprising a thrombolytic metalloproteinase covalently linked to a targeting peptide capable of specifically binding to a component of a thrombus.

2. The thrombolytic agent of claim 1, wherein the metalloproteinase is *Agkistrodon contortrix contortrix* fibrolase (EC 3.4.24.72).

3. The thrombolytic agent of claims 1 or 2,
wherein the peptide is selected from the group consisting of a linear peptide ligand for a GPIIb/IIIa receptor, a cyclic peptide ligand for a GPIIb/IIIa receptor, a peptide ligand for a polymerization site of fibrin, and a peptide comprising an amino acid sequence XGD, wherein X is any amino acid having a sidechain comprising an amino group, a guanidino group, or an amidino group.

4. The thrombolytic agent of claim 3, wherein the peptide is selected from the group consisting of:
HHLGGAKQAGDV (SEQ ID NO:2);
CH₂CO.Y_{D}.Apc.GDCGGC_{Acm}GC_{Acm}GGC.amide (SEQ ID NO:3);
CH₂CO.Y_{D}.Apc.GDCKGC_{Acm}GC_{Acm}GGC.amide (SEQ ID NO:4);
C_{Acm}GC_{Acm}GGRGDSC (SEQ ID NO:5);
C_{Acm}GC_{Acm}GGRGDGGRGDSC (SEQ ID NO:6);
C_{Acm}GC_{Acm}GGRGDGGRGDGGRGDSC (SEQ ID NO:7);
C_{Acm}GC_{Acm}RRRRRRRRRGDVC (SEQ ID NO:8);
CGRGDVKC_{Acm}GC_{Acm}.amide (SEQ ID NO:9);
CGRGDVC_{Acm}GC_{Acm}.amide (SEQ IDNO: 10);
CGRGDVRGDFKC_{Acm}GC_{Acm}.amide (SEQ ID NO:11);
CGRGDVRGDFC_{Acm}GC_{Acm}.amide (SEQ LD NO: 12);
*aceyl*-G.Apc.GDV.Apc.GDFKC_{Acm}GC_{Acm}.GGCamide (SEQ ID NO:13);
G.Apc.GDV.Apc.GDFKC_{Acm}GC_{Acm}.GGC.amide (SEQ IDNO:14);
G.Ape.GDVKC_{Acm}GC_{Acm}GGC.amide (SEQ ID NO:15);
CC_{Acm}GC_{Acm}GGRGDS (SEQ ID NO:16);
*acetyl*-RRARGDDLDC_{Acm}GC_{Acm}GGC.amide (SEQ ID NO:17);
GRGDFGGC_{Acm} (SEQ ID NO:18);
*ma_{Bz}*-GGRGDF (SEQ ID NO:19);
C_{Acm}GGGRGDF (SEQ ID NO:20);
GRGDGGC_{Acm} (SEQ ID NO:21);
*ma*-GGRGDF (SEQ ID NO:22);
*ma_{Acm}*-GGGRGDF (SEQ ID NO:23);
*ma*-RGDF (SEQ ID NO:24);
*ma*-RGD
CH₂CO.Y_{D}.Apc.GDCGGC.amide (SEQ ID NO:25);
CH₂CO.Y_{D}.Apc.GDCGGGC.amide (SEQ ID NO:26);
CH₂CO.Y_{D}.Anc.GDCKGGGC.amide (SEQ ID NO:27);
CH₂CO.Y_{D}.Apc.GDCGGGGC.amide (SEQ ID NO:28);
GGRGDSC (SEQ ID NO:29);
GGRGDGGRGDSC (SEQ ID NO:30);
GGRGDGGRGDGGRGDSC (SEQ ID NO:31);
RRRRRRRRRGDVC (SEQ ID NO:32);
CGRGDVK.amide (SEQ ID NO:33);
CGRGDV.amide (SEQ ID NO:34);
CGRGDVRGDFK.amide (SEQ ID NO:35);
CGRGDVRGDF.amide (SEQ ID NO:36);
*acetyl*-G.Apc.GDV.Apc.GDFKGGCamide (SEQ ID NO:37);
G.Apc.GDV.Apc.GDFKGGCamide (SEQ ID NO:38);
G.Apc.GDVKGGC.amide (SEQ ID NO:39);
CGGRGDS (SEQ ID NO:40);
*acetyl*-RRARGDDLDGGC.amide (SEQ ID NO:41);
CKRARGDDMDDYC (SEQ ID NO:42);
*mmp*-GGGRGDF (SEQ ID NO:43);
*acetyl*-RGDC.amide (SEQ ID NO:44);
CRGDC (SEQ ID NO:45);
CGGGRGDF (SEQ ID NO:46);
GRGDGGGGC (SEQ ID NO:47);
GRGDGGC (SEQ ID NO:48);
*ma_{Acm}*-GGGRGDF (SEQ ID NO:49);
*acetyl*-CNP.Apc.GDC (SEQ ID NO:50);
CRIARGDWNDDYC (SEQ ID NO:51);
CKFFARTVCRIARGDWNDDYCTGKSSDC (SEQ ID NO:52);
KYGGHHLGGAKQAGDV (SEQ ID NO:53);
(CH₂CO.Y_{D}.Apc.GDCKGCG.amide)₂-(ε-K)GC.amide; and
CH₂.CO.Y_{D}.Apc.GDCKGCG.amide (SEQ ID NO:54).

5. The thrombolytic agent of claim 4, wherein the peptide is
CH₂CO.Y_{D}.Apc.GDCKGCG.amide (SEQ ID NO:54).

6. The thrombolytic agent of claims 1 to 5, wherein the compound is covalently linked to the proteinase using a chemical conjugating moiety selected from the group consisting of N-succinimidyl-2-(2-pyridyldithio) propionate, N-(γ-maleimidobutyryloxy) sulfosuccinimide ester (S-GMBS), succinimidyl 4-(*p*-maleimidophenyl)butyrate, sulfosuccinimidyl 4-(*p*-maleimidophenyl)butyrate, 4-succinimidyloxycarbonylmethyl-α-(2-pyridyldithio)toluene, N-hydroxysuccinimidyl-2,3-dibromopropionate, N-succinimidyl-(4-iodoacetyl)-aminobenzoate, sulfosuccinimidyl-(4-iodoacetyl)-aminobenzoate, succinimidyl-4-(N-maleimidomethyl)cyclohexane-1-carboxylate, sulfosuccinimidyl-4-(N-maleimidomethyl)cyclohexane-1-carboxylate, N-(y-maleimidobutyryloxy) succinimide ester, *m*-maleimidobenzenyl-N-hydroxysuccinimide ester, *m*-maleimidobenzenyl-N-hydroxysulfosuccinimide ester, sulfosuccinimidyloxycarbonyl-6-(α-methyl)-α-(2-pyridyldithio) toluamide hexanoate, and sulfosuccinimidyl 6-(α-methyl-α-(2-pyridyldithio) toluamido) hexanoate.

7. The thrombolytic agent of any of claims 3 to 5, produced by expression of a nucleic acid encoding the proteinase and the peptide in a host cell.

8. The thrombolytic agent of claim 1, further comprising a radiolabel.

9. Use of the thrombolytic agent of any of claims 1 to 8 for the manufacture of a pharmaceutical composition for lysing a thrombus within a mammalian body.

## Patentansprüche

1. Thrombolytisches Mittel, enthaltend eine thrombolytische Metalloproteinase kovalent gebunden an ein Targeting-Peptid, das zur spezifischen Bindung an eine Komponente eines Thrombus fähig ist.

2. Thrombolytisches Mittel nach Anspruch 1, wobei es sich bei der Metalloproteinase um *Agkistrodon contortrix contortrix*-Fibrolase (EC 3.4.24.72) handelt.

3. Thrombolytisches Mittel nach Anspruch 1 oder 2, wobei das Peptid ausgewählt ist aus der aus einem geradkettigen Peptidliganden für einen GPIIb/IIIa-Rezeptor, einem cyclischen Peptidliganden für einen GPIIb/IIIa-Rezeptor, einem Peptidliganden für eine Polymerisierungsstelle von Fibrin und einem einer Aminosäuresequenz XGD enthaltenden Peptid bestehenden Gruppe, wobei X für eine Aminosäure mit einer eine Aminogruppe, eine Guanidinogruppe oder eine Amidinogruppe enthaltenden Seitenkette steht.

4. Thrombolytisches Mittel nach Anspruch 3, wobei das Peptid aus der aus:
HHLGGAKQAGDV (SEQ ID NR:2);
CH₂CO.Y_{D}.Apc.GDCGGC_{Acm}GC_{Acm}GGC.Amid (SEQ ID NR:3);
CH₂CO.Y_{D}.Apc.GDCKGC_{Acm}GC_{Acm}GGC.Amid (SEQ ID NR:4);
C_{Acm}GC_{Acm}GGRGDSC (SEQ ID NR:5);
C_{Acm}GC_{Acm}GGRGDGGRGDSC (SEQ ID NR:6);
C_{Acm}GC_{Acm}GGRGDGGRGDGGRGDSC (SEQ ID NR:7);
C_{Acm}GC_{Acm}RRRRRRRRRGDVC (SEQ ID NR:8);
CGRGDVKC_{Acm}GC_{Acm}.Amid (SEQ ID NR:9);
CGRGDVC_{Acm}GC_{Acm}.Amid (SEQ IDNO: 10);
CGRGDVRGDFKC_{Acm}GC_{Acm}.Amid (SEQ ID NR:11);
CGRGDVRGDFC_{Acm}GC_{Acm}.Amid (SEQ ID NR: 12);
*acetyl*-G.Apc.GDV.Apc.GDFKC_{Acm}GC_{Acm}.GGC.Amid (SEQ ID NR:13);
G.Apc.GDV.Apc.GDFKC_{Acm}GC_{Acm}.GGC.Amid (SEQ IDNO: 14);
G.Ape.GDVKC_{Acm}GC_{Acm}GGC.Amid (SEQ ID NR:15);
CC_{Acm}GC_{Acm}GGRGDS (SEQ ID NR:16);
*acetyl*-RRARGDDLDC_{Acm}GC_{Acm}GGC.Amid (SEQ ID NR:17);
GRGDFGGC_{Acm} (SEQ ID NR:18);
*ma_{Bz}*-GGRGDF (SEQ ID NR:19);
C_{Acm}GGGRGDF (SEQ ID NR:20);
GRGDGGC_{Acm} (SEQ ID NR:21);
*ma*-GGRGDF (SEQ ID NR:22);
*ma*_{Acm}-GGGRGDF (SEQ ID NR:23);
*ma*-RGDF (SEQ ID NR:24);
*ma*-RGD;
CH₂CO.Y_{D}.Apc.GDCGGC.Amid (SEQ ID NR:25);
CH₂CO.Y_{D}.Apc.GDCGGGC.Amid (SEQ ID NR:26);
CH₂CO.Y_{D}.Apc.GDCKGGGC.Amid (SEQ ID NR:27);
CH₂CO.Y_{D}.Apc.GDCGGGGC.Amid (SEQ ID NR:28);
GGRGDSC (SEQ ID NR:29);
GGRGDGGRGDSC (SEQ ID NR:30);
GGRGDGGRGDGGRGDSC (SEQ ID NR:31);
RRRRRRRRRGDVC (SEQ ID NR:32);
CGRGDVK.Amid (SEQ ID NR:33);
CGRGDV.Amid (SEQ ID NR:34);
CGRGDVRGDFK.Amid (SEQ ID NR:35);
CGRGDVRGDF.Amid (SEQ ID NR:36);
*acetyl*-G.Apc.GDV.Apc.GDFKGGC.Amid (SEQ ID NR:37);
G.Apc.GDV.Apc.GDFKGGC.Amid (SEQ ID NR:38);
G.Apc.GDVKGGC.Amid (SEQ ID NR:39);
CGGRGDS (SEQ ID NR:40);
*acetyl*-RRARGDDLDGGC.Amid (SEQ ID NR:41);
CKRARGDDMDDYC (SEQ ID NR:42);
*mmp*-GGGRGDF (SEQ ID NR:43);
*acetyl*-RGDC.Amid (SEQ ID NR:44);
CRGDC (SEQ ID NR:45);
CGGGRGDF (SEQ ID NR:46);
GRGDGGGGC (SEQ ID NR:47);
GRGDGGC (SEQ ID NR:48);
*ma*_{Acm}-GGGRGDF (SEQ ID NR:49);
*acetyl*-CNP.Apc.GDC (SEQ ID NR:50);
CRIARGDWNDDYC (SEQ ID NR:51);
CKFFARTVCRIARGDWNDDYCTGKSSDC (SEQ ID NR:52);
KYGGHHLGGAKQAGDV (SEQ ID NR:53);
(CH₂CO-Y_{D}.Amp.GDCKGCG.Amid)₂-(ε-K)GC.Amid und
CH₂CO-Y_{D}.Amp.GDCKGCG.Amid (SEQ ID NR:54)
bestehenden Gruppe ausgewählt ist.

5. Das thrombolytische Mittel nach Anspruch 4, wobei es sich bei dem Peptid um CH₂CO-Y_{D}.Amp.GDCKGCG.Amid (SEQ ID NR:54) handelt.

6. Thrombolytisches Mittel nach einem der Ansprüche 1 bis 5, wobei die Verbindung mit einer chemisch konjugierenden Einheit ausgewählt aus der aus N-Succinimidyl-2-(2-pyridyldithio)propionat, N-(γ-Maleimidobutyryloxy)sulfosuccinimidester, Succinimidyl-4-(p-maleimidophenyl)butyrat, Sulfosuccinimidyl-4-(p-maleimidophenyl)butyrate, 4-Succinimidyloxycarbonylmethyl-α-(2-pyridyldithio)toluol, N-Hydroxysuccinimidyl-2,3-dibrompropionat, N-Succinimidyl-(4-iodacetyl)aminobenzoat, Sulfosuccinimidyl-(4-iodacetyl)aminobenzoat, Succinimidyl-4-(N-maleimidomethyl)cyclohexan-1-carboxylat, Sulfosuccinimidyl-4-(N-maleimidomethyl)cyclohexan-1-carboxylate, N-(γ-Maleimidobutyryloxy)succinimidester, *m-*Maleimidobenzenyl-N-hydroxysuccinimidester, *m*-Maleimidobenzenyl-N-hydroxysulfosuccinimidester, Sulfosuccinimidyloxycarbonyl-6-(α-methyl)-α-(2-pyridyldithio)toluamidhexanoat und Sulfosuccinimidyl-6-(α-methyl-a-(2-pyridyldithio)toluamido)hexanoat bestehenden Gruppe kovalent an die Proteinase gebunden ist.

7. Thrombolytisches Mittel nach einem der Ansprüche 3 bis 5, hergestellt durch Expression einer die Proteinase und das Peptid in einer Wirtszelle exprimierenden Nukleinsäure.

8. Thrombolytisches Mittel nach Anspruch 1, weiterhin enthaltend einen radioaktiven Marker.

9. Verwendung des thrombolytischen Mittels nach einem der Ansprüche 1 bis 8 zur Herstellung einer pharmazeutischen Zusammensetzung zur Lyse eines Thrombus in einem Säugetierkörper.

## Revendications

1. Agent thrombolitique comprenant une métallo-protéinase thrombolitique liée de manière covalente à un peptide cible susceptible de se lier de manière spécifique à un composant d'un thrombus.

2. Agent thrombolitique selon la revendication 1, **caractérisé en ce que** la métalloprotéinase est la fibrolase d'*Agkistrodon contortrix contortrix* (EC 3.4.24.72).

3. Agent thrombolitique selon la revendication 1 ou 2, **caractérisé en ce que** le peptide est choisi dans le groupe constitué d'un ligand peptidique linéaire d'un récepteur de GPIIb/IIIa, d'un ligand peptidique cyclique d'un récepteur de GPIIb/IIIa, d'un ligand peptidique pour un site de polymérisation de la fibrine et d'un peptide comprenant une séquence d'acides aminés XGD, où X est un acide aminé quelconque ayant une chaîne latérale comprenant un groupement amino, un groupement guanidino ou un groupement amidino.

4. Agent thrombolitique selon la revendication 3, **caractérisé en ce que** le peptide est choisi dans le groupe constitué de :
HHLGGAKQAGDV (SEQ ID N° 2);
CH₂CO.Y_{D}.Apc.GDCGGC_{Acm}GC_{Acm}GGC.amide (SEQ ID N° 3);
CH₂CO.Y_{D}.Apc.GDCKGC_{Acm}GC_{Acm}GGC.amide (SEQ ID N° 4);
C_{Acm}GC_{Acm}GGRGDSC (SEQ ID N° 5);
C_{Acm}GC_{Acm}GGRGDGGRGDSC (SEQ ID N° 6);
C_{Acm}GC_{Acm}GGRGDGGRGDGGRGDSC (SEQ ID N° 7);
C_{Acm}GC_{Acm}RRRRRRRRRGDVC (SEQ ID N° 8);
CGRGDVKC_{Acm}GC_{Acm}.amide (SEQ ID N° 9);
CGRGDVC_{Acm}GC_{Acm}.amide (SEQ ID N° 10);
CGRGDVRGDFKC_{Acm}GC_{Acm}.amide (SEQ ID N° 11);
CGRGDVRGDFC_{Acm}GC_{Acm}.amide (SEQ ID N° 12);
*acétyl*-G.Apc.GDV.Apc.GDFKC_{Acm}GC_{Acm}.GGC.amide (SEQ ID N° 13);
G.Apc.GDV.Apc.GDFKC_{Acm}GC_{Acm}.GGC.amide (SEQ ID N° 14);
G.Ape.GDVKC_{Acm}GC_{Acm}GGC.amide (SEQ ID N° 15);
CC_{Acm}GC_{Acm}GGRGDS (SEQ ID N° 16);
*acétyl*-RRARGDDLDC_{Acm}GC_{Acm}GGC,amide (SEQ ID N° 17);
GRGDFGGC_{Acm} (SEQ ID N° 18);
*ma_{Bz}*-GGRGDF (SEQ ID N° 19);
C_{Acm}GGGRGDF (SEQ ID N° 20);
GRGDGGC_{Acm} (SEQ ID N° 21);
*ma*-GGRGDF (SEQ ID N° 22);
*ma*_{Acm}-GGGRGDF (SEQ ID N° 23);
*ma*-RGDF (SEQ ID N° 24);
*ma*-RGD;
CH₂CO.Y_{D}.Apc.GDCGGC.amide (SEQ ID N° 25);
CH₂CO.Y_{D}.Apc.GDCGGGC.amide (SEQ ID N° 26);
CH₂CO.Y_{D}.Apc.GDCKGGGC.amide (SEQ ID N° 27);
CH₂CO.Y_{D}.Apc.GDCGGGGC.amide (SEQ ID N° 28);
GGRGDSC (SEQ ID N° 29);
GGRGDGGRGDSC (SEQ ID N° 30);
GGRGDGGRGDGGRGDSC (SEQ ID N° 31);
RRRRRRRRRGDVC (SEQ ID N° 32);
CGRGDVK.amide (SEQ ID N° 33);
CGRGDV.amide (SEQ ID N° 34);
CGRGDVRGDFK.amide (SEQ ID N° 35);
CGRGDVRGDF.amide (SEQ ID N° 36);
*acétyl*-G.Apc.GDV.Apc.GDFKGGCamide (SEQ ID N° 37);
G.Apc.GDV.Apc.GDFKGGCamide (SEQ ID N° 38);
G.Apc.GDVKGGC.amide (SEQ ID N° 39);
CGGRGDS (SEQ ID N° 40);
*acétyl*-RRARGDDLDGGC.amide (SEQ ID N° 41);
CKRARGDDMDDYC (SEQ ID N° 42);
*mmp*-GGGRGDF (SEQ ID N° 43);
*acétyl*-RGDC.amide (SEQ ID N° 44);
CRGDC (SEQ ID N° 45);
CGGGRGDF (SEQ ID N° 46);
GRGDGGGGC (SEQ ID N° 47);
GRGDGGC (SEQ ID N° 48);
*ma*_{Acm}-GGGRGDF (SEQ ID N° 49);
*acétyl*-CNP.Apc.GDC (SEQ ID N° 50);
CRIARGDWNDDYC (SEQ ID N° 51);
CKFFARTVCRIARGDWNDDYCTGKSSDC (SEQ ID N° 52);
KYGGHHLGGAKQAGDV (SEQ ID N° 53);
(CH₂CO-Y_{D}.Apc.GDCKGCG.amide)₂-(ε-K)GC.amide; et
CH₂CO-Y_{D}.Apc.GDCKGCG.amide (SEQ ID N° 54).

5. Agent thrombolitique selon la revendication 4, **caractérisé en ce que** le peptide est :
CH₂CO-Y_{D}.Apc.GDCKGCG.amide (SEQ ID N° 54).

6. Agent thrombolitique selon les revendications 1 à 5, **caractérisé en ce que** le composé est lié de manière covalente à la protéinase à l'aide d'un motif de conjugaison chimique choisi dans le groupe constitué de
N-succinimidyl-2-(2-pyridyldithio)propionate,
ester de N-(γ-maléimidobutyryloxy)sulfosuccinimide (S-GMBS),
4-(*p*-maléimidophényl)butyrate de succinimidyle,
4-(*p*-maléimidophényl)butyrate de sulfo-succinimidyle,
4-succinimidyloxycarbonylméthyl-α-(2-pyridyldithio)toluène,
N-hydroxysuccinimidyl-2,3-dibromopropionate,
N-succinimidyl-(4-iodoacétyl)aminobenzoate,
sulfosuccinimidyl-(4-iodoacétyl)aminobenzoate,
succinimidyl-4-(N-maléimidométhyl)cyclohexane-1-carboxylate,
sulfosuccinimidyl-4-(N-maléimidométhyl)cyclohexane-1-carboxylate,
ester de N-(γ-maléimidobutyryloxy)succinimide,
ester de *m*-maléimidobenzényl-N-hydroxysuccinimide,
ester de *m*-maléimidobenzényl-N-hydroxysulfosuccinimide,
sulfosuccinimidyloxycarbonyl-6-(α-méthyl)-α-(2-pyridyldithio)toluamide
hexanoate et
6-(α-méthyl-α-(2-pyridyldithio)toluamido)hexanoate de sulfosuccinimidyle.

7. Agent thrombolitique selon l'une quelconque des revendications 3 à 5 produit par l'expression d'un acide nucléide codant pour la protéinase et le peptide dans une cellule hôte.

8. Agent thrombolitique selon la revendication 1 comprenant en outre un radiomarqueur.

9. Utilisation de l'agent thrombolitique de l'une quelconque des revendications 1 à 8, pour la fabrication d'une composition pharmaceutique destinée à la lyse d'un thrombus dans un corps de mammifère.
